# EUROPEAN PATENT APPLICATION

(11) **EP 4 365 184 A1**
(43) Date of publication of application: **08.05.2024**
(21) Application number: 22833331.6
(22) Date of filing: 01.07.2022
(51) Int. Cl.: C07K 1/04, C07K 1/16, C07K 2/00, B01D 15/32, B01D 15/40, B01D 15/42, G01N 30/02, G01N 30/26, G01N 30/88

(54) **ANALYSIS METHOD FOR PEPTIDE BOUND TO CARRIER FOR LIQUID PHASE PEPTIDE SYNTHESIS**

(30) Priority: 02.07.2021 JP 2021111127; 15.12.2021 JP 2021203703
(71) Applicant: Peptistar Inc., Settsu-shi, Osaka 566-0022 (JP); Sekisui Medical Co., Ltd., Tokyo 103-0027 (JP)
(72) Inventor: NEMOTO, Yoshitaka, Settsu-shi, Osaka 566-0022 (JP); ABE, Hitoshi, Tokyo 103-0027 (JP); MORI, Toshihiro, Tokyo 103-0027 (JP)
(74) Representative: Wächtershäuser & Hartz Patentanwaltspartnerschaft mbB
(86) International application number: PCT/JP2022/026477
(87) International publication number: WO 2023/277186

(57) **Abstract**

To provide a means capable of simultaneously and accurately analyzing a component derived from a carrier for liquid phase peptide synthesis with, for example, a target peptide.

A method for simultaneously analyzing a carrier for liquid phase peptide synthesis, a component derived from the carrier for liquid phase peptide synthesis, an amino acid to which the carrier for liquid phase peptide synthesis is bound, and a peptide compound to which the carrier for liquid phase peptide synthesis is bound, with a target peptide or final target peptide, the analysis method using high-performance liquid chromatography or supercritical fluid chromatography using an alcohol as an eluent.

## Description

### Technical Field

The present invention relates to an analysis method for a peptide bound to a carrier for liquid phase peptide synthesis.

### Background Art

As a peptide production technique, there are a solid phase peptide synthesis method and a liquid phase peptide synthesis method, but a liquid phase peptide synthesis method suitable for mass production is widely adopted in order to produce a peptide used, for example, as a pharmaceutical product. Recently, a carrier for liquid phase peptide synthesis (Tag), which is a compound greatly improving the solubility of a protected amino acid or a protected peptide in an organic solvent, has been reported (Patent Literatures 1 to 14).

The liquid phase peptide synthesis with the carrier for liquid phase peptide synthesis has an advantage that the reaction rate of each step can be analyzed because a condensation reaction and a deprotection reaction can be performed in an organic solvent. High-performance liquid chromatography (HPLC) is used for such reaction rate or quality control, side reaction control, and impurity analysis. Non Patent Literature 1 discloses a technique for analyzing a peptide compound synthesized with a carrier for liquid phase peptide synthesis by HPLC analysis with an acetonitrile-based eluent. The substance analyzed in this literature was a peptide obtained after cleaving the carrier for liquid phase peptide synthesis. Non Patent Literature 2 discloses a technique for analyzing the reaction rate of a peptide extension reaction with nanostar, which is a carrier for liquid phase peptide synthesis, in an acetonitrile-based solvent in a state where the nanostar is bound. However, the analysis in Non Patent Literature 2 is a technique for analyzing substances to which a carrier for liquid phase peptide synthesis is bound, and it is silent on a technique for simultaneously analyzing both a substance to which a carrier for liquid phase peptide synthesis is bound and a substance to which a carrier for liquid phase peptide synthesis is not bound. Non Patent Literature 3 discloses a technique in which after a binding reaction between Trt(OH)-K10, which is a carrier for liquid phase peptide synthesis, and a peptide is performed, the remaining amount of Trt(OH)-K10 and the peptide and production of the peptide to which Trt(OH)-K10 is bound are analyzed by HPLC with an acetonitrile-based eluent.

### Citation List

### Patent Literatures

Patent Literature 1: JP 5113118 B2
Patent Literature 2: JP 5929756 B2
Patent Literature 3: JP 6092513 B2
Patent Literature 4: JP 5768712 B2
Patent Literature 5: JP 5803674 B2
Patent Literature 6: JP 6116782 B2
Patent Literature 7: JP 6201076 B2
Patent Literature 8: JP 6283774 B2
Patent Literature 9: JP 6283775 B2
Patent Literature 10: JP 6322350 B2
Patent Literature 11: JP 6393857 B2
Patent Literature 12: JP 6531235 B2
Patent Literature 13: WO 2020/175472 A
Patent Literature 14: WO 2020/175473 A

### Non Patent Literatures

Non Patent Literature 1: Angew Chem Int Ed Engl. 2017, 56, 7803-7807
Non Patent Literature 2: Angew Chem Int Ed Engl. 2021, 60, 7786-7795.
Non Patent Literature 3: Angew Chem Int Ed Engl. 2018, 57, 2105-2109.

### Summary of Invention

### Technical Problem

Analysis of a pharmaceutical product is strictly defined for impurity control as in "IMPURITIES IN NEW DRUG SUBSTANCES among new active ingredient-containing pharmaceutical products", and for example, it is necessary to perform scientific evaluation of impurities predicted to be present or to accurately analyze the amount of impurities by setting an extremely small amount of 0.05% as a threshold value. Therefore, when multiple types of impurities are analyzed, it is common to set multiple analysis methods in accordance with characteristics of respective impurities.

If a carrier for liquid phase peptide synthesis, a component derived from the carrier for liquid phase peptide synthesis that is by-produced under conditions after deprotection of the carrier for liquid phase peptide synthesis, or an amino acid or peptide compound to which the carrier for liquid phase peptide synthesis is bound can be simultaneously analyzed by HPLC with a peptide obtained after the carrier is cleaved for liquid phase peptide synthesis, the reaction rate or quality control, and side reaction control can be easy as described above. However, the carrier for liquid phase peptide synthesis, the component derived from the carrier for liquid phase peptide synthesis that is by-produced under conditions after deprotection of the carrier for liquid phase peptide synthesis, or the amino acid or peptide compound to which the carrier for liquid phase peptide synthesis is bound has high lipid solubility, and may not be dissolved in an acetonitrile-based eluent generally used in HPLC analysis. The peptide to which the carrier for liquid phase peptide synthesis is bound is adsorbed on the HPLC column stationary phase, and it may be difficult to elute the peptide with an acetonitrile-based eluent. At the time of deprotection, the polarity of the carrier for liquid phase peptide synthesis or the component derived therefrom is greatly different from the polarity of an intended peptide component. Therefore, since the carrier for liquid phase peptide synthesis or the component derived therefrom may not be analyzed in the analysis conditions for measuring the quality of the peptide, in order to confirm the amount of the carrier for liquid phase peptide synthesis or the component derived therefrom in a sample, it is necessary to separately examine the analysis conditions and perform HPLC analysis in individual analysis conditions for each detection component. That is, when the amount of the carrier for liquid phase peptide synthesis or the component derived therefrom was analyzed in one condition, the peptide component was not separated from other impurities or not eluted, and when the amount of the peptide component was analyzed in the other condition, the carrier for liquid phase peptide synthesis or the component derived therefrom was not separated from other impurities or not eluted.

Meanwhile, although it was possible to purify the peptide obtained after cleaving the carrier for liquid phase peptide synthesis by combining solid-liquid separation and reverse phase high-performance liquid chromatography, the purification is intended to isolate a required amount of a target compound at a quality equal to or higher than a target purity, and for example, contaminants other than the target compound may not be separated from each other, or may not be adsorbed and eluted on the HPLC column stationary phase. Therefore, the purification may not be used as an analysis method that requires quantitative or qualitative observation of the content or content ratio of each of the target compound and the contaminants.

Therefore, the present invention aims to provide a means capable of analyzing a carrier for liquid phase peptide synthesis, a component derived from the carrier for liquid phase peptide synthesis that is by-produced under conditions after deprotection of the carrier for liquid phase peptide synthesis, or an amino acid or peptide compound to which the carrier for liquid phase peptide synthesis is bound and simultaneously and accurately analyzing these compounds with, for example, a peptide obtained after cleaving the carrier for liquid phase peptide synthesis (sometimes referred to as "target peptide" hereinafter), a peptide obtained by deprotecting an N-terminal or C-terminal protective group from the target peptide, or a peptide obtained after further deprotecting a general protective group, which is not a carrier for liquid phase synthesis, from the peptide obtained by deprotecting an N-terminal or C-terminal protective group (sometimes referred to as "final target peptide" hereinafter).

### Solution to Problem

The present inventors have conducted various investigations to find a means capable of simultaneously analyzing components having greatly different polarities such as a carrier for liquid phase peptide synthesis, a component derived from the carrier for liquid phase peptide synthesis that is by-produced under conditions after deprotection of the carrier for liquid phase peptide synthesis, or an amino acid or peptide compound to which the carrier for liquid phase peptide synthesis is bound, with a target peptide or final target peptide. As a result, the inventors found that these components can be analyzed with an alcohol instead of acetonitrile as an eluent for liquid chromatography such as HPLC, and these compounds can be simultaneously and accurately analyzed with, for example, a target peptide, thereby completing the present invention.

That is, the present invention provides the following inventions [1] to [7].
[1] A method for simultaneously analyzing one or more compounds selected from the group consisting of a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound, with a target peptide or final target peptide, the analysis method using high-performance liquid chromatography or supercritical fluid chromatography using, as an eluent, a solution containing an alcohol having 1 to 4 carbon atoms,
   wherein the carrier for liquid phase peptide synthesis being a compound of Formula (I) below: 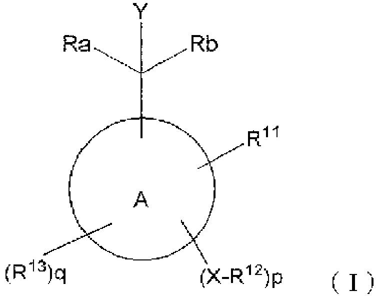
   wherein ring A represents a C4-20 aromatic ring which may contain a hetero atom and may be polycyclic;
   R¹¹ is a hydrogen atom, or when ring A is a benzene ring and Rb is a group of Formula (a) below, represents a single bond together with R¹⁴, and may form a fluorene ring together with ring A and ring B or may form a xanthene ring together with ring A and ring B via an oxygen atom;
   p X's each independently represent -O-, -S-, - C(=O)O-, -C(=O)NH-, -NHC(=O)-, or -NR¹⁷- (R¹⁷ represents a hydrogen atom, an alkyl group, or an aralkyl group); and
   p R¹²'s each independently represent an organic group of an aliphatic hydrocarbon group substituted with an aliphatic hydrocarbon group via an oxygen atom, or Formula (b) ;

      **-R²⁰-X³-D** (b)
   provided that R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound;
   q R¹³'s each independently are a hydrogen atom or represent an organic group having an aliphatic hydrocarbon group which may be substituted with a silyl group or an aliphatic hydrocarbon group via an oxygen atom;
   p and q each represent an integer of from 0 to 4 and p + q is 1 or more and 4 or less;
   ring A may further have, in addition to p XR¹²'s, a substituent selected from the group consisting of a halogen atom, a C1-6 alkyl group which may be substituted with a halogen atom, and a C1-6 alkoxy group which may be substituted with a halogen atom;
   Ra represents a hydrogen atom or an aromatic ring which may be substituted with a halogen atom; and
   Rb represents a hydrogen atom, an aromatic ring which may be substituted with a halogen atom, or a group of Formula (a): 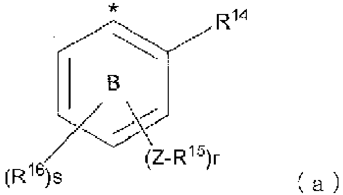
   wherein, * represents a bonding position;
   r and s each represent an integer of from 0 to 4 and r + s is 4 or less;
   r Z's each independently represent -O-, -S-, - C(=O)O-, -C(=O)NH-, -NHC(=O)-, or -NR¹⁸- (R¹⁸ represents a hydrogen atom, an alkyl group, or an aralkyl group); and
   r R¹⁵'s independently represent an organic group of an aliphatic hydrocarbon group substituted with an aliphatic hydrocarbon group, or Formula (b);

      **-R²⁰-X³-D** (b)
   provided that R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound;
   s R¹⁶'s each independently represent an organic group having an aliphatic hydrocarbon group which may be substituted with a silyl group or an aliphatic hydrocarbon group via an oxygen atom;
   R¹⁴ represents a hydrogen atom, or represents a single bond together with R¹¹, and may form a fluorene ring together with ring A and ring B, or may form a xanthene ring together with ring A and ring B via an oxygen atom; and
   ring B may further have, in addition to r ZR¹⁵'s, a substituent selected from the group consisting of a halogen atom, a C1-6 alkyl group which may be substituted with a halogen atom, and a C1-6 alkoxy group which may be substituted with a halogen atom; and
   Y represents a hydroxy group, a thiol group, NHR¹⁹ (R¹⁹ represents a hydrogen atom, an alkyl group, or an aralkyl group), or a halogen atom.
[2] A method for simultaneously analyzing one or more compounds selected from the group consisting of a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound, with a target peptide or final target peptide, the analysis method using high-performance liquid chromatography or supercritical fluid chromatography using, as an eluent, a solution containing an alcohol having 1 to 4 carbon atoms,
   wherein the carrier for liquid phase peptide synthesis being a compound of Formula (1), (13), or (14) below: 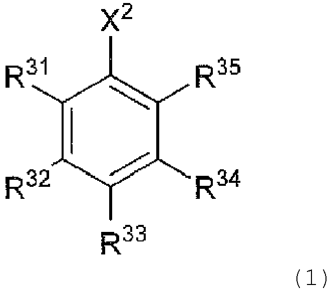
   wherein X² represents -CH₂OR⁴⁴ (R⁴⁴ represents a hydrogen atom, a halogenocarbonyl group, an active ester-type carbonyl group, or an active ester-type sulfonyl group), -CH₂NHR⁴⁵ (R⁴⁵ represents a hydrogen atom, a linear or branched alkyl group having 1 to 6 carbon atoms, or an aralkyl group), a halogenomethyl group, a formyl group, or an oxime, and at least one of R³¹, R³², R³³, R³⁴, and R³⁵ represents a group of Formula (c),

      **-O-R²⁰-X³-D** (c)
   and the rest represents a hydrogen atom, a halogen atom, an alkyl group having 1 to 4 carbon atoms, or an alkoxy group having 1 to 4 carbon atoms;
   R²⁰ represents a linear or branched alkylene group having 6 to 16 carbon atoms;
   X³ represents O or CONR²¹ (R²¹ represents a hydrogen atom or an alkyl group having 1 to 4 carbon atoms); and
   D represents a group of Formula (2), (3), (4), (5), (6), (7), (8), (9), (10), (11), or (12): 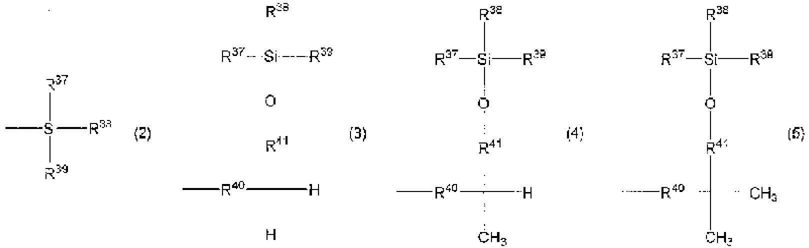 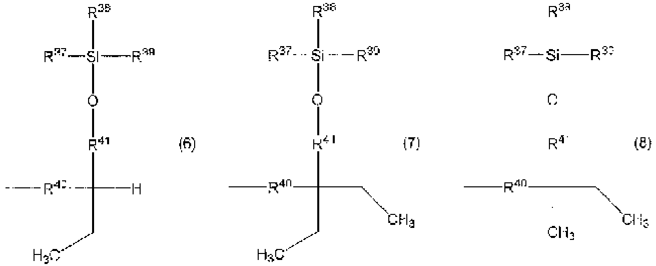 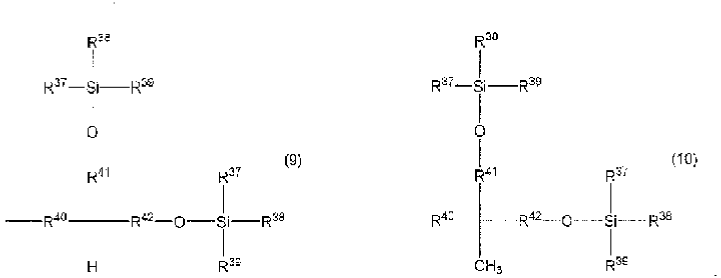 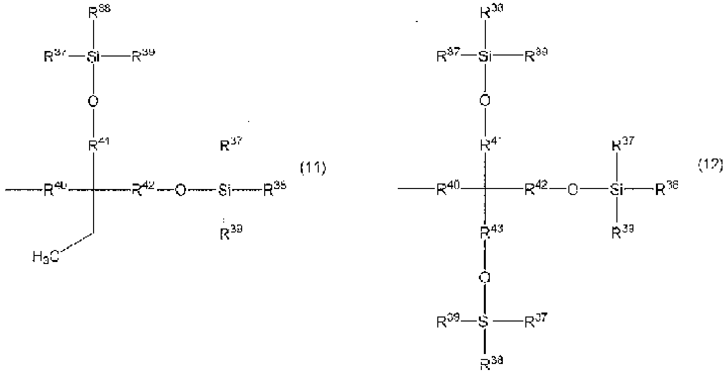
   wherein R³⁷, R³⁸, and R³⁹ are the same or different and each represent a linear or branched alkyl group having 1 to 6 carbon atoms or an aryl group which may have a substituent; R⁴⁰ represents a single bond or a linear or branched alkylene group having 1 to 3 carbon atoms; and R⁴¹, R⁴², and R⁴³ each represent a linear or branched alkylene group having 1 to 3 carbon atoms), 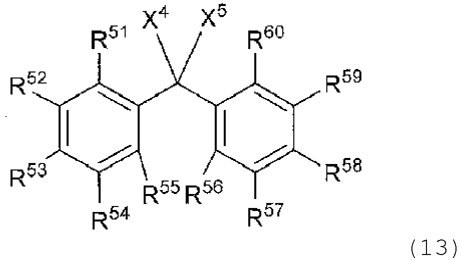
   wherein X⁴ represents -OR⁶¹ (R⁶¹ represents a hydrogen atom, an active ester-type carbonyl group, or an active ester-type sulfonyl group), -NHR⁴⁵, azide, halogen, isocyanate, or =N-OH or =O together with X⁵, when X⁴ is - OR⁶¹, -NHR⁴⁵, azide, or halogen, X⁵ represents a hydrogen atom, a linear or branched alkyl group or alkenyl group having 1 to 4 carbon atoms, or a cycloalkyl group, and when X⁴ is isocyanate, X⁵ represents a linear or branched alkyl group or alkenyl group having 1 to 4 carbon atoms, or a cycloalkyl group;
   at least one of R⁵¹ to R⁶⁰ represents a group of Formula (c), and the rest represents a hydrogen atom, a halogen atom, an alkyl group having 1 to 4 carbon atoms, or an alkoxy group having 1 to 4 carbon atoms; and
   when X⁴ is -OR⁶¹, -NHR⁴⁵, azide, or halogen and X⁵ is a hydrogen atom, or when X⁴ is =O together with X⁵, R⁵⁵ and R⁵⁶ may be bound via an oxygen atom to form a xanthene ring; 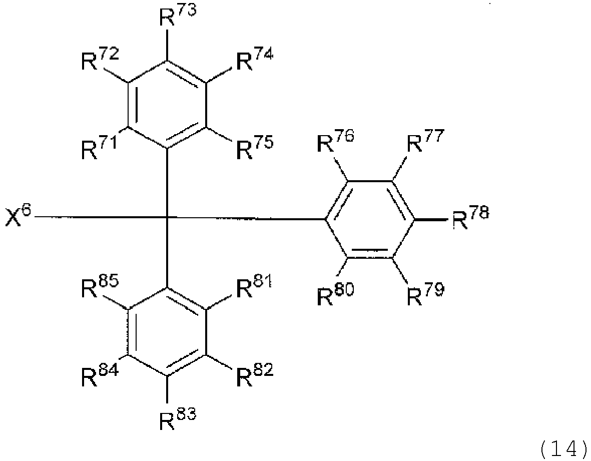
   wherein X⁶ represents a hydroxy group or a halogen atom, at least one of R⁷¹ to R⁸⁵ represents a group of Formula (c), the rest represents a hydrogen atom, a halogen atom, an alkyl group having 1 to 4 carbon atoms, or an alkoxy group having 1 to 4 carbon atoms, and R⁸⁰ and R⁸¹ may be bound by a single bond to form a fluorene ring, or may be bound via an oxygen atom to form a xanthene ring.
[3] A method for simultaneously analyzing one or more compounds selected from the group consisting of a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound, with a target peptide or final target peptide, the analysis method using high-performance liquid chromatography or supercritical fluid chromatography using, as an eluent, a solution containing an alcohol having 1 to 4 carbon atoms,
   wherein the carrier for liquid phase peptide synthesis being a compound of Formula (20) or (21) below: 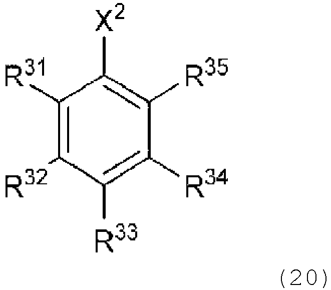
   wherein X² represents -CH₂OR⁴⁴ (R⁴⁴ represents a hydrogen atom, a halogenocarbonyl group, an active ester-type carbonyl group, or an active ester-type sulfonyl group), -CH₂NHR⁴⁵ (R⁴⁵ represents a hydrogen atom, a linear or branched alkyl group having 1 to 6 carbon atoms, or an aralkyl group), a halogenomethyl group, a formyl group, or an oxime, and at least one of R³¹, R³², R³³, R³⁴, and R³⁵ represents a group of Formula (d),

      **-O-R²⁰-X³-E** (d)
   and the rest represents a hydrogen atom, a halogen atom, an alkyl group having 1 to 4 carbon atoms, or an alkoxy group having 1 to 4 carbon atoms;
   R²⁰ represents a linear or branched alkyl group or alkylene group having 6 to 30 carbon atoms;
   X³ is absent or represents an oxygen atom; and
   E is absent or represents a linear or branched alkylene group having 6 to 30 carbon atoms: 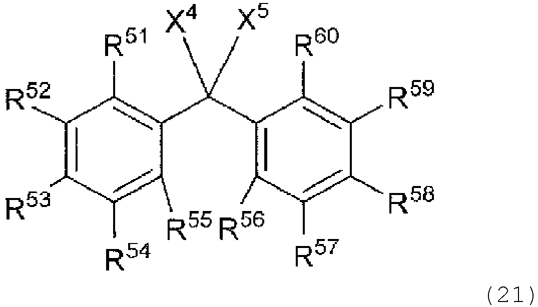
   wherein X⁴ represents a hydroxyl group or -NHR⁹⁰ (R⁹⁰ represents a hydrogen atom, a linear or branched alkyl group having 1 to 6 carbon atoms, or an aralkyl group), and X⁵ represents a hydrogen atom or a phenyl group which may be substituted with a halogen atom; and
   at least one of R⁵¹ to R⁶⁰ represents a group of Formula (d), and the rest represents a hydrogen atom, a halogen atom, an alkyl group having 1 to 4 carbon atoms, or an alkoxy group having 1 to 4 carbon atoms.
[4] The analysis method according to any one of [1] to [3], wherein the carrier for liquid phase peptide synthesis is a compound which is bound to an amino acid, a peptide, or an amino acid amide directly or via a linker to make the amino acid, the peptide, or the amino acid amide soluble in an organic solvent and insoluble in water.
[5] The analysis method according to any one of [1] to [4], wherein a sample used for analysis is a reaction solution in which an object to be analyzed is not subjected to solid-liquid separation.
[6] The analysis method according to any one of [1] to [4], wherein a sample used for analysis is a sample obtained after subjecting an object to be analyzed to solid-liquid separation or purification by chromatography.
[7] The method according to any one of [1] to [6], wherein one or more detected or undetected compounds selected from the group consisting of a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound are linked with a detected target peptide or a detected final target peptide to perform evaluation.

### Advantageous Effects of Invention

According to the present invention, it is possible to solve the problem in that an amino acid, peptide, or amino acid amide to which the carrier for liquid phase peptide synthesis is bound (sometimes referred to as "peptide bound to a carrier for liquid phase peptide synthesis" hereinafter) is not dissolved in the eluent and the problem in that the peptide bound to a carrier for liquid phase peptide synthesis is not eluted, and it is possible to simultaneously analyze components having greatly different polarities such as a carrier for liquid phase peptide synthesis, a component derived from the carrier for liquid phase peptide synthesis that is by-produced under conditions after deprotection of the carrier for liquid phase peptide synthesis, or an amino acid or peptide compound to which the carrier for liquid phase peptide synthesis is bound, with a target peptide or final target peptide. In HPLC, by adding an acidic compound to the eluent, the reaction rate of each step can be grasped by analysis using exactly the same eluent in a series of peptide synthesis. Since the analysis of the peptide component can be simultaneously performed, it is also possible to determine that the component derived from the carrier for liquid phase peptide synthesis is not mixed in the target peptide or final target peptide after deprotection.

### Brief Description of Drawings

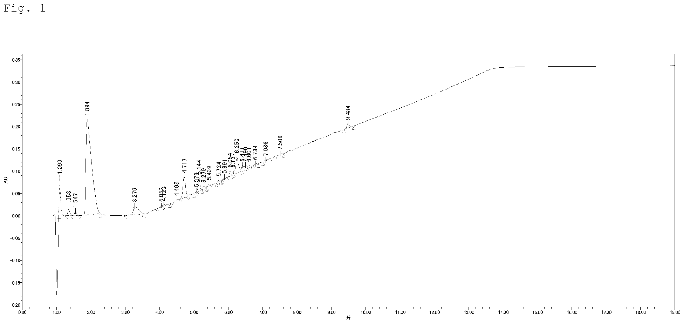

Fig. 1 shows HPLC analysis results obtained by analyzing H-Ser-Met-Ile-Leu-Glu-OH synthesized in Example 4 in Analysis Condition 1.

### Description of Embodiments

An embodiment of the present invention is a method for analyzing one or more compounds selected from the group consisting of a carrier for liquid phase peptide synthesis, a component derived from the carrier for liquid phase peptide synthesis, an amino acid to which the carrier for liquid phase peptide synthesis is bound, and a peptide compound to which the carrier for liquid phase peptide synthesis is bound, the analysis method using high-performance liquid chromatography or supercritical fluid chromatography using an alcohol as an eluent. As a preferred embodiment of the analysis method of the present invention, there is mentioned a method for simultaneously analyzing one or more compounds selected from the group consisting of a carrier for liquid phase peptide synthesis, a component derived from the carrier for liquid phase peptide synthesis, an amino acid to which the carrier for liquid phase peptide synthesis is bound, and a peptide compound to which the carrier for liquid phase peptide synthesis is bound, with a target peptide or final target peptide, the method using high-performance liquid chromatography or supercritical fluid chromatography using an alcohol as an eluent.

As an embodiment of the analysis method of the present invention, there is mentioned an analysis method in which reaction tracking or post-treatment tracking of each step is performed in a liquid phase peptide production method using one or more compounds selected from the group consisting of an amino acid to which a carrier for liquid phase peptide synthesis is bound and a peptide compound to which a carrier for liquid phase peptide synthesis is bound, the analysis method using high-performance liquid chromatography or supercritical fluid chromatography using an alcohol as an eluent.

A more preferred embodiment of the present invention is a method for analyzing a carrier for liquid phase peptide synthesis or a compound derived from the carrier for liquid phase peptide synthesis, the analysis method including subjecting a sample containing a target peptide or final target peptide obtained in an arbitrary step of a liquid phase peptide production method using a compound to which a carrier for liquid phase peptide synthesis and an amino acid, peptide, or amino acid amide are bound or obtained by the production method to high-performance liquid chromatography or supercritical fluid chromatography using an alcohol as an eluent.

In the present specification, the term "target peptide" refers to a peptide obtained by a condensation reaction using an amino acid or peptide to which the carrier for liquid phase peptide synthesis is bound, and refers to a peptide obtained after cleaving the carrier for liquid phase peptide synthesis. The target peptide is a peptide from which the carrier for liquid phase peptide synthesis is cleaved and which has a protective group at the N-terminal or C-terminal. A functional group of an amino acid (amino acid residue) side chain such as a hydroxyl group, an amino group, a carboxyl group, or a thiol group may be protected by a general protective group that is not a carrier for liquid phase synthesis such as a tBu group.

The final target peptide refers to a peptide obtained by deprotecting an N-terminal or C-terminal protective group from the target peptide or a peptide obtained after further deprotecting a general protective group, which is not a carrier for liquid phase synthesis, from the peptide obtained by deprotecting an N-terminal or C-terminal protective group.

The analysis means that each component contained in a substance to be analyzed is separated and the content or content ratio thereof is quantitatively or qualitatively observed.

Tracking refers to observing the degree of progress of a chemical reaction linked with the lapse of time, and means observing, for example, a decrease in starting materials related to the chemical reaction and an increase in products related to the chemical reaction. More specifically, the tracking refers to quantitatively or qualitatively observing the content of the amino acid or peptide having an amino group protected by an amino protective group (sometimes referred to as "amino group-protected amino acid" hereinafter), the content of the amino acid or peptide to which the carrier for liquid phase peptide synthesis is bound, the content of the carrier for liquid phase peptide synthesis or the component derived from the carrier for liquid phase peptide synthesis, and the content of the target peptide or final target peptide to which the carrier for liquid phase peptide synthesis is bound.

Simultaneously analyzing refers to observing two or more desired components by one injection of a sample into an HPLC column. More specifically, simultaneously analyzing refers to quantitatively or qualitatively observing the content of a target peptide or final target peptide and the content of a carrier for liquid phase peptide synthesis or a component derived from the carrier for liquid phase peptide synthesis by injecting a sample once into an HPLC column.

The expression "linked to perform evaluation" refers to, for example, where the purity is calculated based on the ratio of the contents of the detected target peptide or detected final target peptide and one or more detected or undetected compounds selected from the group consisting of a carrier for liquid phase peptide synthesis, a component derived from the carrier for liquid phase peptide synthesis, an amino acid to which the carrier for liquid phase peptide synthesis is bound, and a peptide compound to which the carrier for liquid phase peptide synthesis is bound.

A sample to be analyzed by the analysis method of the present invention is a sample containing a target peptide or final target peptide obtained in an arbitrary step of the liquid phase peptide production method using a compound in which a carrier for liquid phase peptide synthesis and an amino acid, peptide, or amino acid amide are bound or obtained by the method. The sample contains one or more carriers for liquid phase peptide synthesis, a component derived from the carrier for liquid phase peptide synthesis that is by-produced under conditions after deprotection of the carrier for liquid phase peptide synthesis, an amino acid or peptide to which the carrier for liquid phase peptide synthesis is bound, a target peptide, and a final target peptide. Specific examples of these samples include a reaction solution in which an object to be analyzed is not subjected to solid-liquid separation and a sample obtained after subjecting an object to be analyzed to solid-liquid separation or purification by chromatography. In the conventional method, as a matter of course, in the case of using a reaction solution in which an object to be analyzed is not subjected to solid-liquid separation, even when a sample obtained after subjecting an object to be analyzed to solid-liquid separation or purification by chromatography is used as a sample, it is difficult to simultaneously analyze a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, a peptide compound to which the carrier for liquid phase peptide synthesis is bound, with a target peptide or final target peptide, which are embodiments of the present invention, particularly to simultaneously analyze a carrier for liquid phase peptide synthesis and a final target peptide, and simultaneous analysis using the sample is one of preferred embodiments of the present invention.

According to the present invention, with respect to the composition containing a target peptide or final target peptide, it is possible to determine whether or not a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound are mixed or the mixing ratio, simultaneously with analysis of a target peptide or final target peptide.

First, the liquid phase peptide production method using a compound in which a carrier for liquid phase peptide synthesis and an amino acid, peptide, or amino acid amide are bound that can suitably use the analysis method of the present invention will be described.

The liquid phase peptide production method is preferably a liquid phase peptide production method including the following steps a to e of:
a. binding a carrier for liquid phase peptide synthesis to an amino acid, a peptide, an amino acid amide, or an amino acid or peptide having an amino group protected by an amino protective group in a solvent containing an organic solvent;
b. condensing the amino acid, peptide, or amino acid amide to which the carrier for liquid phase peptide synthesis is bound, and the amino acid or peptide having an amino group protected by an amino protective group in the solvent containing an organic solvent;
c. adding an amino acid active ester scavenger to a reaction solution after the condensation reaction;
d. deprotecting the amino protective group of the compound having an amino group protected by an amino protective group in the reaction solution; and
e. adding an aqueous solution to the reaction solution and performing liquid separation to obtain an organic solvent layer containing a condensate of the amino acid, peptide, or amino acid amide to which the carrier for liquid phase peptide synthesis is bound and the amino acid or peptide from which the amino protective group is deprotected.

The carrier for liquid phase peptide synthesis used in the step a is preferably a compound which is bound to an amino acid, a peptide, or an amino acid amide directly or via a linker to make the amino acid, the peptide, or the amino acid amide soluble in an organic solvent and insoluble in water.

Examples of such a carrier for liquid phase peptide synthesis include the compounds described in Patent Literatures 1 to 14 described above. Preferable examples of the carrier for liquid phase peptide synthesis include a compound of Formula (I) below: 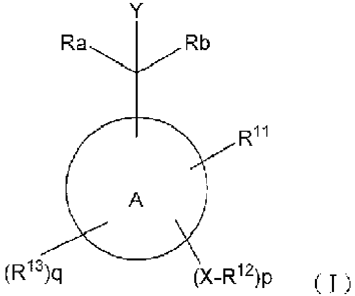
wherein ring A represents a C4-20 aromatic ring which may contain a hetero atom and may be polycyclic;
R¹¹ is a hydrogen atom, or when ring A is a benzene ring and Rb is a group of Formula (a) below, represents a single bond together with R¹⁴, and may form a fluorene ring together with ring A and ring B or may form a xanthene ring together with ring A and ring B via an oxygen atom;
p X's each independently represent -O-, -S-, - C(=O)O-, -C(=O)NH-, -NHC(=O)-, or -NR¹⁷- (R¹⁷ represents a hydrogen atom, an alkyl group, or an aralkyl group); and
p R¹²'s each independently represent an organic group of an aliphatic hydrocarbon group substituted with an aliphatic hydrocarbon group via an oxygen atom, or Formula (b) ;

   **-R²⁰-X³-D** (b)
provided that R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound;
q R¹³'s each independently are a hydrogen atom or represent an organic group having an aliphatic hydrocarbon group which may be substituted with a silyl group or an aliphatic hydrocarbon group via an oxygen atom;
p and q each represent an integer of 0 to 4 and p + q is 1 or more and 4 or less;
ring A may further have, in addition to p XR¹²'s, a substituent selected from the group consisting of a halogen atom, a C1-6 alkyl group which may be substituted with a halogen atom, and a C1-6 alkoxy group which may be substituted with a halogen atom;
Ra represents a hydrogen atom or an aromatic ring which may be substituted with a halogen atom; and
Rb represents a hydrogen atom, an aromatic ring which may be substituted with a halogen atom, or a group of Formula (a): 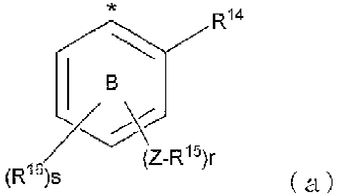
wherein * represents a bonding position;
r and s each represent an integer of 0 to 4 and r + s is 4 or less;
r Z's each independently represent -O-, -S-, - C(=O) O-, -C(=O)NH-, -NHC(=O)-, or -NR¹⁸- (R¹⁸ represents a hydrogen atom, an alkyl group, or an aralkyl group); and
r R¹⁵'s independently represent an organic group of an aliphatic hydrocarbon group substituted with an aliphatic hydrocarbon group, or Formula (b);

   **-R²⁰-X³-D** (b)
provided that R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound;
s R¹⁶'s each independently represent an organic group having an aliphatic hydrocarbon group which may be substituted with a silyl group or an aliphatic hydrocarbon group via an oxygen atom;
R¹⁴ represents a hydrogen atom, or represents a single bond together with R¹¹, and may form a fluorene ring together with ring A and ring B, or may form a xanthene ring together with ring A and ring B via an oxygen atom; and
ring B may further have, in addition to r ZR¹⁵'s, a substituent selected from the group consisting of a halogen atom, a C1-6 alkyl group which may be substituted with a halogen atom, and a C1-6 alkoxy group which may be substituted with a halogen atom; and
Y represents a hydroxy group, a thiol group, NHR¹⁹ (R¹⁹ represents a hydrogen atom, an alkyl group, or an aralkyl group), or a halogen atom.
Ring A in Formula (I) represents a C4-20 aromatic ring which may contain a hetero atom and may be monocyclic or polycyclic. Examples of the aromatic ring include a C6-20 aromatic hydrocarbon ring and a C4-10 aromatic heterocyclic ring.

Specific examples of the C6-20 aromatic hydrocarbon ring include a benzene ring, a naphthalene ring, an anthracene ring, a phenanthrene ring, a triphenylene ring, a tetracene ring, an indane ring, an indene ring, a fluorene ring, a biphenyl ring, and a 1,1'-binaphthalene ring. Among them, a benzene ring, a naphthalene ring, a phenanthrene ring, a fluorene ring are more preferable. A benzene ring is most preferable.

The C4-10 aromatic heterocyclic ring is preferably a 5- to 10-membered aromatic heterocyclic ring containing one to three atoms selected from the group consisting of a nitrogen atom, an oxygen atom, and a sulfur atom as a hetero atom, and specific examples thereof include a pyrrole ring, a furan ring, a thiophene ring, an indole ring, a benzofuran ring, a benzothiophene ring, a carbazole ring, a pyrazole ring, an indazole ring, an imidazole ring, a pyridine ring, a quinoline ring, and an isoquinoline ring. Among them, a 5- to 8-membered aromatic heterocyclic ring containing one to three atoms selected from the group consisting of a nitrogen atom, an oxygen atom, and a sulfur atom as a hetero atom is preferable, and a pyrrole ring, a furan ring, a thiophene ring, an indole ring, a benzofuran ring, a benzothiophene ring, a carbazole ring, a pyrazole ring, and an indazole ring are more preferable.

R¹¹ is a hydrogen atom, or when ring A is a benzene ring and Rb is a group of Formula (a) above, represents a single bond together with R¹⁴, and may form a fluorene ring together with ring A and ring B or may form a xanthene ring together with ring A and ring B via an oxygen atom. As the ring which R¹¹ may form together with R¹⁴ is preferably a fluorene ring or a xanthene ring.

p X's each independently represent -O-, -S-, - C(=O)O-, -C(=O)NH-, -NHC(=O)-, or -NR¹⁷- (R¹⁷ represents a hydrogen atom, an alkyl group, or an aralkyl group). -O-, -S-, -C(=O)O-, -C(=O)NH-, or -NR¹⁷- is preferable, -O- or - NR¹⁷- is further preferable, and -O- or -NH is most preferable.

R¹⁷ is preferably a hydrogen atom, a C1-10 alkyl group, or a C7-20 aralkyl group. Examples of the alkyl group include linear or branched C1-10 alkyl groups such as a methyl group, an ethyl group, a propyl group, an isopropyl group, a n-butyl group, an isobutyl group, a sec-butyl group, a tert-butyl group, a pentyl group, and a hexyl group.

Examples of the aralkyl group include C7-16 aralkyl groups such as a benzyl group, a 1-phenylethyl group, a 2-phenylethyl group, a 1-phenylpropyl group, a naphthylmethyl group, and a 1-naphthylethyl group.
p R¹²'s each independently represent an organic group of an aliphatic hydrocarbon group substituted with an aliphatic hydrocarbon group via an oxygen atom, or Formula (b) ;

   **-R²⁰-X³-D** (b)
provided that, R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound;
q R¹³'s each independently are a hydrogen atom or represent an organic group having an aliphatic hydrocarbon group which may be substituted with a silyl group or an aliphatic hydrocarbon group via an oxygen atom.
p and q each represent an integer of 0 to 4 and p + q represents 1 or more and 4 or less.

In the present specification, the organic group having an aliphatic hydrocarbon group is a monovalent organic group having an aliphatic hydrocarbon group in its molecular structure. The site of the aliphatic hydrocarbon group in the organic group having an aliphatic hydrocarbon group is not particularly limited, and may be present at the terminal or may be present at other sites.

The aliphatic hydrocarbon group present in the organic group is a linear, branched, or cyclic saturated or unsaturated aliphatic hydrocarbon group, and is preferably a C5 or higher aliphatic hydrocarbon group, more preferably a C5-30 aliphatic hydrocarbon group, and further preferably a C8-30 aliphatic hydrocarbon group, from the viewpoint of organic solvent solubility. Specific examples of the aliphatic hydrocarbon group include an alkyl group, a cycloalkyl group, an alkenyl group, and an alkynyl group, and in particular, an alkyl group, a cycloalkyl group, and an alkenyl group are preferable, and an alkyl group is more preferable. A C5-30 linear or branched alkyl group, a C3-8 cycloalkyl group, and a C5-30 linear or branched alkenyl group are preferable, a C5-30 linear or branched alkyl group and a C3-8 cycloalkyl group are more preferable, a C5-30 linear or branched alkyl group is further preferable, and a C8-30 linear or branched alkyl group is still more preferable.

Specific examples of the alkyl group include alkyl groups having 1 to 30 carbon atoms, and examples thereof include monovalent groups such as a methyl group, an ethyl group, a propyl group, an isopropyl group, a butyl group, an isobutyl group, a sec-butyl group, a tert-butyl group, a pentyl group, a hexyl group, an octyl group, a decyl group, a lauryl group, a tridecyl group, a myristyl group, a cetyl group, a stearyl group, an aralkyl group, a behenyl group, a tetracosanyl group, a hexacosanyl group, and an isostearyl group, divalent groups derived therefrom, and groups obtained by excluding, for example, a hydroxyl group from various steroid groups.

Examples of the alkenyl group include monovalent groups such as a vinyl group, a 1-propenyl group, an allyl group, an isopropenyl group, a butenyl group, an isobutenyl group, and an oleyl group, and divalent groups derived therefrom.

Examples of the alkynyl group include an ethynyl group, a propargyl group, and a 1-propynyl group.

In the aliphatic hydrocarbon group, an aliphatic hydrocarbon group may be substituted via an oxygen atom. Examples of the aliphatic hydrocarbon group that can be substituted with an aliphatic hydrocarbon group via an oxygen atom include monovalent groups such as a linear or branched alkoxy group having 1 to 20 carbon atoms, an alkenyloxy group having 2 to 20 carbon atoms, and a cycloalkyloxy group having 3 to 6 carbon atoms, and divalent groups derived therefrom. The aliphatic hydrocarbon group in which an aliphatic hydrocarbon group is substituted via an oxygen atom may further have a repeating structure in which an aliphatic hydrocarbon group is substituted via an oxygen atom.

Specific examples of R¹² include a 12-docosyloxy-1-dodecyl group, a 3,4,5-tris(octadecyloxy)benzyl group, a 2,2,2-tris(octadecyloxymethyl)ethyl group, and a 3,4,5-tris(octadecyloxy)cyclohexylmethyl group. These three kinds are preferable.

In the aliphatic hydrocarbon group, a silyl group or an aliphatic hydrocarbon group may be substituted via an oxygen atom, and examples of the substituent include a group of the following Formula (b).

**-R²⁰-X³-D** (b)

R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound.

The silyl group is preferably a silyl group in which one to three alkyl groups or one to three aryl groups are substituted, and more preferably a silyl group in which three linear or branched alkyl groups having 1 to 6 carbon atoms are substituted or a silyl group in which three alkyl groups having 6 to 10 carbon atoms which may have a substituent are substituted. Examples of the aryl group include a phenyl group and a naphthyl group.

The silyl group is preferably a silyl group in which three linear or branched alkyl groups having 1 to 6 carbon atoms are substituted, and more preferably a silyl group in which three linear or branched alkyl groups having 1 to 4 carbon atoms are substituted. The three alkyl groups or aryl groups substituted for the silyl group may be the same or different.

The alkyl group to which a silyloxy group is bound is preferably a linear or branched alkyl group having 1 to 13 carbon atoms to which one to three silyloxy groups are bound in which three groups selected from the group consisting of a linear or branched alkyl group having 1 to 6 carbon atoms and an aryl group which may have a substituent are substituted. The silyloxy group is preferably a silyloxy group in which three linear or branched alkyl groups having 1 to 6 carbon atoms are substituted, and more preferably a silyloxy group in which three linear or branched alkyl groups having 1 to 4 carbon atoms are substituted. The three alkyl groups or aryl groups substituted for the silyloxy group may be the same or different.

The linear or branched alkyl group having 1 to 13 carbon atoms is preferably branched, and more preferably has a quaternary carbon atom.

p and q each represent an integer of 0 to 4 and p + q represents 1 or more and 4 or less. p is preferably 1 to 4, more preferably 1 to 3, and further preferably 1 to 2.

Ring A may further have, in addition to p XR¹²'s, a substituent selected from the group consisting of a halogen atom, a C1-6 alkyl group which may be substituted with a halogen atom, and a C1-6 alkoxy group which may be substituted with a halogen atom.

Examples of the halogen atom include a chlorine atom, a fluorine atom, a bromine atom, and an iodine atom. Examples of the C1-6 alkyl group which may be substituted with a halogen atom include a methyl group, an ethyl group, a propyl group, an isopropyl group, a butyl group, an isobutyl group, a sec-butyl group, a tert-butyl group, a pentyl group, a hexyl group, a dichloromethyl group, a trichloromethyl group, and a trifluoromethyl group. Examples of the C1-6 alkoxy group which may be substituted with a halogen atom include a methoxy group, an ethoxy group, a propyloxy group, an isopropyloxy group, a butyloxy group, an isobutyloxy group, a sec-butyloxy group, a tert-butyloxy group, a trichloromethoxy group, and a trifluoromethoxy group.

Ra represents a hydrogen atom or an aromatic ring which may be substituted with a halogen atom.

Examples of the aromatic ring include a C6-18 aromatic hydrocarbon ring and a C4-10 aromatic heterocyclic ring.

Specific examples of the C6-18 aromatic hydrocarbon ring include a benzene ring, a naphthalene ring, an anthracene ring, a phenanthrene ring, a triphenylene ring, a tetracene ring, an indane ring, an indene ring, a fluorene ring, and a biphenyl ring. Among them, a benzene ring, a naphthalene ring, a phenanthrene ring, a fluorene ring are more preferable. A benzene ring is most preferable.

The C4-10 aromatic heterocyclic ring is preferably a 5- to 10-membered heterocyclic ring containing one to three atoms selected from the group consisting of a nitrogen atom, an oxygen atom, and a sulfur atom as a hetero atom, and specific examples thereof include a pyrrole ring, a furan ring, a thiophene ring, an indole ring, a benzofuran ring, a benzothiophene ring, a carbazole ring, a pyrazole ring, an indazole ring, an imidazole ring, a pyridine ring, a quinoline ring, and an isoquinoline ring. Among them, a 5- to 8-membered heterocyclic ring containing one to three atoms selected from the group consisting of a nitrogen atom, an oxygen atom, and a sulfur atom as a hetero atom is preferable, and a pyrrole ring, a furan ring, a thiophene ring, an indole ring, a benzofuran ring, a benzothiophene ring, a carbazole ring, a pyrazole ring, and an indazole ring are more preferable.

One to three halogen atoms may be substituted in the aromatic ring of Ra.

Rb represents a hydrogen atom, an aromatic ring which may be substituted with a halogen atom, or the group of Formula (a).

In Formula (a), r and s each represent an integer of 0 to 4 and r + s is 0 to 4.

r is preferably 0 to 4, more preferably 1 to 3, and further preferably 1 to 2.

r Z's each independently represent -O-, -S-, - C(=O)O-, -C(=O)NH-, -NHC(=O)-, or -NR¹⁸- (R¹⁸ represents a hydrogen atom, an alkyl group, or an aralkyl group). -O-, -S-, -C(=O)O-, -C(=O)NH-, or -NR¹⁷- is preferable, -O- or - NR¹⁷- is further preferable, and -O- or -NH is most preferable.

R¹⁸ is preferably a hydrogen atom, a C1-10 alkyl group, or a C7-20 aralkyl group. Examples of the alkyl group include a methyl group, an ethyl group, a propyl group, an isopropyl group, a n-butyl group, an isobutyl group, a sec-butyl group, a tert-butyl group, a pentyl group, and a hexyl group.

Examples of the aralkyl group include C7-16 aralkyl groups such as a benzyl group, a 1-phenylethyl group, a 2-phenylethyl group, a 1-phenylpropyl group, a naphthylmethyl group, and a 1-naphthylethyl group.

r R¹⁵'s independently represent an organic group of an aliphatic hydrocarbon group substituted with an aliphatic hydrocarbon group via an oxygen atom, or Formula (b) ;

-**R²⁰-X³-D** (b)

provided that R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound.
s R¹⁶'s each independently represent an organic group having an aliphatic hydrocarbon group which may be substituted with a silyl group or an aliphatic hydrocarbon group via an oxygen atom.

Examples of the organic group having an aliphatic hydrocarbon group which may be substituted with a silyl group or an aliphatic hydrocarbon group via an oxygen atom represented as R¹⁵ and R¹⁶ include the same groups as those of R¹² and R¹³ described above, and the same groups as those of R¹² and R¹³ described above are preferable.

R¹⁴ represents a hydrogen atom, or represents a single bond together with R¹¹, and may form a fluorene ring together with ring A and ring B, or may form a xanthene ring together with ring A and ring B via an oxygen atom.

Ring B may further have, in addition to r ZR¹⁵'s, a substituent selected from the group consisting of a halogen atom, a C1-6 alkyl group which may be substituted with a halogen atom, and a C1-6 alkoxy group which may be substituted with a halogen atom.

Examples of the halogen atom include a chlorine atom, a fluorine atom, a bromine atom, and an iodine atom. Examples of the C1-6 alkyl group which may be substituted with a halogen atom include a methyl group, an ethyl group, a propyl group, an isopropyl group, a butyl group, an isobutyl group, a sec-butyl group, a tert-butyl group, a pentyl group, a hexyl group, a dichloromethyl group, a trichloromethyl group, and a trifluoromethyl group. Examples of the C1-6 alkoxy group which may be substituted with a halogen atom include a methoxy group, an ethoxy group, a propyloxy group, an isopropyloxy group, a butyloxy group, an isobutyloxy group, a sec-butyloxy group, a tert-butyloxy group, a trichloromethoxy group, and a trifluoromethoxy group.

Y represents a hydroxy group, a thiol group, NHR¹⁹ (R¹⁹ represents a hydrogen atom, an alkyl group, or an aralkyl group), or a halogen atom.

R¹⁹ is preferably a hydrogen atom, a C1-10 alkyl group, or a C7-20 aralkyl group. Examples of the alkyl group include a methyl group, an ethyl group, a propyl group, an isopropyl group, a n-butyl group, an isobutyl group, a sec-butyl group, a tert-butyl group, a pentyl group, and a hexyl group.

Examples of the aralkyl group include C7-16 aralkyl groups such as a benzyl group, a 1-phenylethyl group, a 2-phenylethyl group, a 1-phenylpropyl group, a naphthylmethyl group, and a 1-naphthylethyl group.

Among the compounds of Formula (I), specific examples of a preferred carrier for liquid phase peptide synthesis include compounds of Formula (1), (13), or (14) below. As one example thereof, a compound of Formula (1) can be used (Patent Literatures 6 and 7).

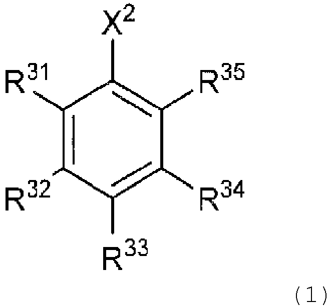

Wherein X² represents -CH₂OR⁴⁴ (R⁴⁴ represents a hydrogen atom, a halogenocarbonyl group, an active ester-type carbonyl group, or an active ester-type sulfonyl group), -CH₂NHR⁴⁵ (R⁴⁵ represents a hydrogen atom, a linear or branched alkyl group having 1 to 6 carbon atoms, or an aralkyl group), a halogenomethyl group, a formyl group, or an oxime, and at least one of R³¹, R³², R³³, R³⁴, and R³⁵ represents a group of Formula (c),

**-O-R²⁰-X³-D** (c)

and the rest represents a hydrogen atom, a halogen atom, an alkyl group having 1 to 4 carbon atoms, or an alkoxy group having 1 to 4 carbon atoms;
R²⁰ represents a linear or branched alkylene group having 6 to 16 carbon atoms;
X³ represents O or CONR²¹ (R²¹ represents a hydrogen atom or an alkyl group having 1 to 4 carbon atoms); and
D represents a group of Formula (2), (3), (4), (5), (6), (7), (8), (9), (10), (11), or (12): 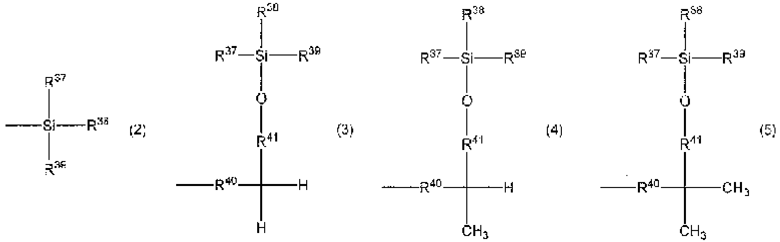 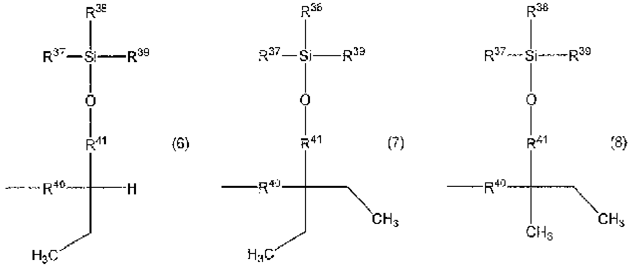 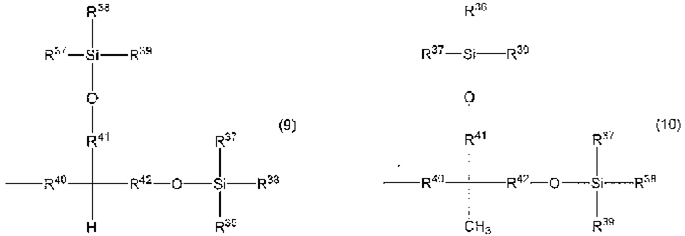 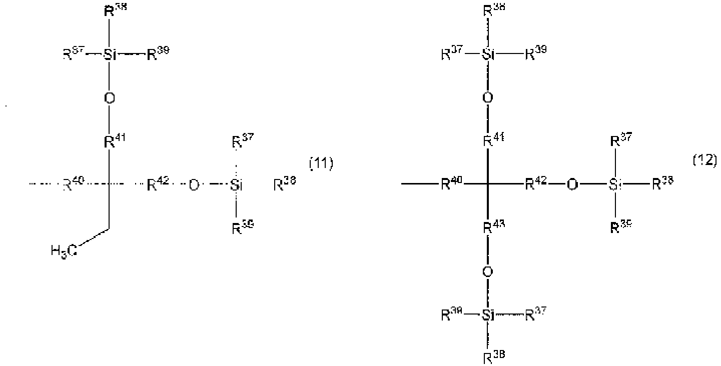
wherein R³⁷, R³⁸, and R³⁹ are the same or different and each represent a linear or branched alkyl group having 1 to 6 carbon atoms or an aryl group which may have a substituent; R⁴⁰ represents a single bond or a linear or branched alkylene group having 1 to 3 carbon atoms; and R⁴¹, R⁴², and R⁴³ each represent a linear or branched alkylene group having 1 to 3 carbon atoms.

As the carrier for liquid phase peptide synthesis, a compound of Formula (13) can be used (Patent Literatures 8, 9, and 12). 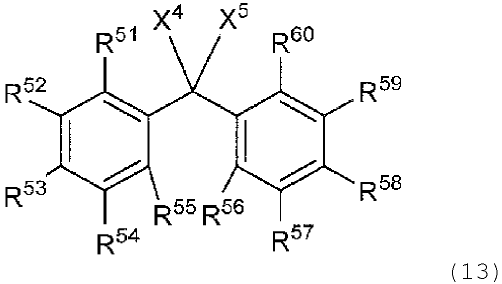
Wherein X⁴ represents -OR⁶¹ (R⁶¹ represents a hydrogen atom, an active ester-type carbonyl group, or an active ester-type sulfonyl group), -NHR⁴⁵, azide, halogen, isocyanate, or =N-OH or =O together with X⁵, when X⁴ is - OR⁶¹, -NHR⁴⁵, azide, or halogen, X⁵ represents a hydrogen atom, a linear or branched alkyl group or alkenyl group having 1 to 4 carbon atoms, or a cycloalkyl group, and when X⁴ is isocyanate, X⁵ represents a linear or branched alkyl group or alkenyl group having 1 to 4 carbon atoms, or a cycloalkyl group;
at least one of R⁵¹ to R⁶⁰ represents a group of Formula (c), and the rest represents a hydrogen atom, a halogen atom, an alkyl group having 1 to 4 carbon atoms, or an alkoxy group having 1 to 4 carbon atoms; and
when X⁴ is -OR⁶¹, -NHR⁴⁵, azide, or halogen and X⁵ is a hydrogen atom, or when X⁴ is =O together with X⁵, R⁵⁵ and R⁵⁶ may be bound via an oxygen atom to form a xanthene ring.

As the carrier for liquid phase peptide synthesis, a compound of Formula (14) can be used (Patent Literatures 10 and 11).

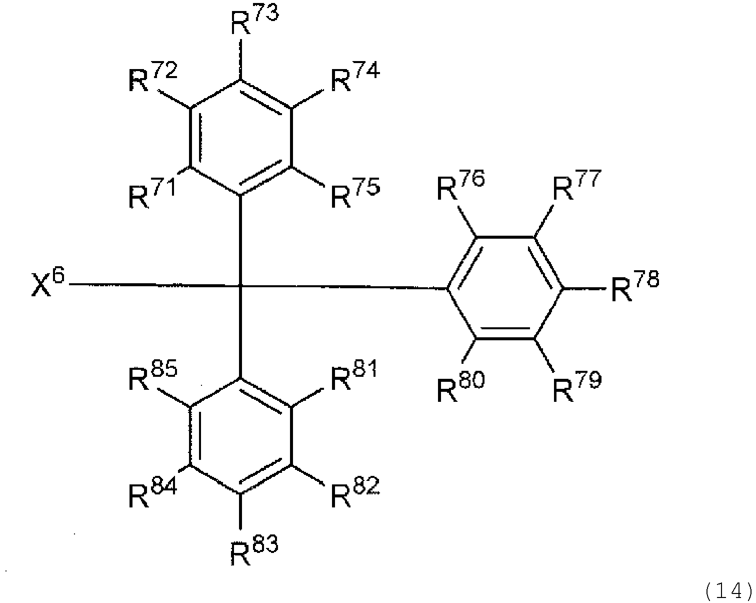

Wherein X⁶ represents a hydroxy group or a halogen atom, at least one of R⁷¹ to R⁸⁵ represents a group of Formula (c), the rest represents a hydrogen atom, a halogen atom, an alkyl group having 1 to 4 carbon atoms, or an alkoxy group having 1 to 4 carbon atoms, and R⁸⁰ and R⁸¹ may be bound by a single bond to form a fluorene ring, or may be bound via an oxygen atom to form a xanthene ring.

Among the compounds of Formula (I), specific examples of a preferred carrier for liquid phase peptide synthesis include compounds of Formula (20) or (21) below. As one example thereof, a compound of Formula (20) can be used (Patent Literatures 2 and 5).

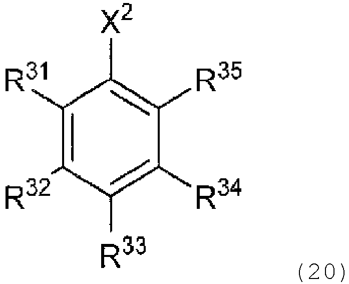

Wherein X² represents a hydroxyl group or -NHR⁹⁰ (R⁹⁰ represents a hydrogen atom, a linear or branched alkyl group having 1 to 6 carbon atoms, or an aralkyl group), at least one of R³¹, R³², R³³, R³⁴, and R³⁵ represents a group of Formula (d),

-O-R²⁰-X³-E (d)

and the rest represents a hydrogen atom, a halogen atom, an alkyl group having 1 to 4 carbon atoms, or an alkoxy group having 1 to 4 carbon atoms;
R²⁰ represents a linear or branched alkyl group or alkylene group having 6 to 30 carbon atoms;
X³ is absent or represents an oxygen atom; and
E is absent or represents a linear or branched alkylene group having 6 to 30 carbon atoms.

As the carrier for liquid phase peptide synthesis, a compound of Formula (21) can be used (Patent Literatures 3 and 4).

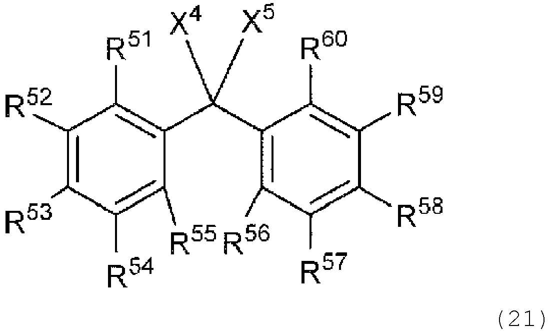

Wherein X⁴ represents a hydroxyl group or -NHR⁹⁰ (R⁹⁰ represents a hydrogen atom, a linear or branched alkyl group having 1 to 6 carbon atoms, or an aralkyl group), and X⁵ represents a hydrogen atom or a phenyl group which may be substituted with a halogen atom; and
at least one of R⁵¹ to R⁶⁰ represents a group of Formula (d), and the rest represents a hydrogen atom, a halogen atom, an alkyl group having 1 to 4 carbon atoms, or an alkoxy group having 1 to 4 carbon atoms.

The amino acid to which the carrier for liquid phase peptide synthesis is bound or the peptide to which the carrier for liquid phase peptide synthesis is bound, which is one of raw materials used in the production method of the present invention, refers to an amino acid or peptide in which one of reactive groups of the amino acid or peptide is bound to the carrier for liquid phase peptide synthesis and at least one amino group is in a reactive state. Preferably, the carboxyl group of the amino acid or peptide is bound to the carrier for liquid phase peptide synthesis, while the amino group is unprotected and reactive. The amino acid amide bound to the carrier for liquid phase peptide synthesis refers to an amino acid amide in which at least one amide group of the amino acid amide is bound to the carrier for liquid phase peptide synthesis, and at least one amino group is unprotected and reactive.

When the amino acid, peptide, or amino acid amide to which the carrier for liquid phase peptide synthesis is bound has a highly reactive functional group such as a hydroxyl group, an amino group, a guanidyl group, a carboxyl group, a thiol group, an indole group, or an imidazole group, a general protective group used in peptide synthesis may be introduced into these functional groups, and a target compound can be obtained by deprotecting the protective group as necessary after completion of the reaction. In this case, examples of the protective group of the hydroxyl group include a tBu group, a Trt group, a Bz group, an acetyl group, and a silyl group, examples of the protective group of the amino group include a Boc group, a Fmoc group, a Cbz group, a Trt group, an Mmt group, and an ivDde group, examples of the protective group of the guanidyl group include a Pbf group, a Pmc group, and a nitro group, examples of the protective group of the carboxyl group include a tBu group, a methyl group, an ethyl group, and a Bz group, examples of the protective group of the thiol group include a Trt group, an Acm group, a tBu group, and an S-tBu group, examples of the protective group of the indole group include a Boc group, and examples of the protective group of the imidazole group include a Boc group, a Bom group, a Bum group, and a Trt group.

The carrier for liquid phase peptide synthesis is introduced so as to be bound to the carboxyl group of the amino acid or peptide directly or via a linker.

The linker described herein is an organic group having two reactive groups in which one of linkers is bound to the carboxyl group and the other is bound to the carrier for liquid phase peptide synthesis. A preferred linker is a compound which is an organic group having a molecular weight of about 2000 or less (preferably about 1500 or less, more preferably about 1000 or less), may be the same or different as the reactive group, and is a compound having at least two groups selected from the group consisting of an amino group, a carboxyl group, and a halomethyl group in the molecule. Examples thereof include the following compounds: 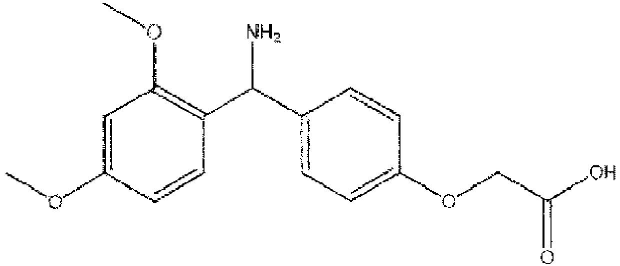 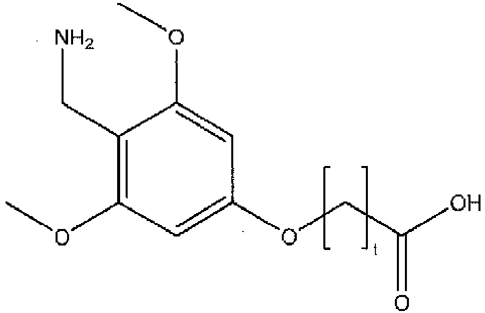 wherein t is an integer of 1 to 6, preferably 1 to 4; 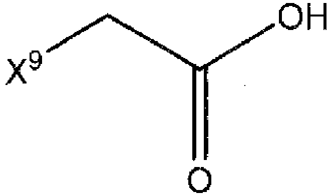 wherein X⁹ is a halogen atom and preferably chlorine or bromine; 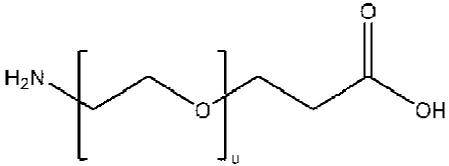
wherein u is an integer of 2 to 40, preferably 2 to 35, more preferably 2 to 28; and
in the structural formula of the linker shows a state before the linker is bound to, for example, a carboxyl group and a state before the linker is bound to the carrier for liquid phase peptide synthesis.

In the introduction of the carrier for liquid phase peptide synthesis including the linker into the carboxyl group, one of the linkers may be bound to the carboxyl group and then the other may be bound to the carrier for liquid phase peptide synthesis, or one of the linkers may be bound to the carrier for liquid phase peptide synthesis and then the other may be bound to the carboxyl group. A means for introducing these linkers into the carboxyl group can be appropriately referred to a known method. For example, amidation with diisopropylcarbodiimide (DIPCI)/HOBt can be mentioned. The binding between one of the linkers and the carrier for liquid phase peptide synthesis can be performed by appropriately referring to a known method depending on the groups of the linkers to be bound to each other and the group of the carrier for liquid phase peptide synthesis. For example, esterification with DIPCI can be mentioned.

As for the amino acid, peptide, or amino acid amide to which the carrier for liquid phase peptide synthesis is bound, an N-Boc-liquid phase synthesis carrier-protected amino acid, peptide, or amino acid amide, which is an intermediate in which the carrier for liquid phase peptide synthesis is bound to the carboxyl group of the amino acid, peptide, or amino acid amide, can be produced by dissolving the carrier for liquid phase peptide synthesis in an organic solvent such as THF and performing condensation by adding, for example, a Boc-protected amino acid, peptide, or amino acid amide and a condensing agent such as DIPCI.

The amino acid or peptide having an amino group protected by an amino protective group, which is the other raw material, means an amino acid or peptide in which the amino group of the amino acid or peptide is protected by an amino protective group, while the carboxyl group is not protected, and which is reactive. When the amino acid or peptide has one or more amino groups, at least one amino group may be protected by an amino protective group.

Examples of the amino protective group include an Fmoc group, a Boc group, a Cbz group, and an Ac group, and among them, an Fmoc group that can be deprotected under basic conditions is more preferable.

When the amino acid or peptide having an amino group protected by an amino protective group has a highly reactive functional group such as a hydroxyl group, an amino group, a guanidyl group, a carboxyl group, a thiol group, an indole group, or an imidazole group, a general protective group used in peptide synthesis may be introduced into these functional groups, and a target compound can be obtained by deprotecting the protective group as necessary at any time point after completion of the reaction.

Examples of the protective group of the hydroxyl group include a tBu group, a Trt group, a Bz group, an acetyl group, and a silyl group, examples of the protective group of the amino group include a Boc group, a Fmoc group, a Cbz group, a Trt group, an Mmt group, and an ivDde group, examples of the protective group of the guanidyl group include a Pbf group, a Pmc group, and a nitro group, examples of the protective group of the carboxyl group include a tBu group, a methyl group, an ethyl group, and a Bz group, examples of the protective group of the thiol group include a Trt group, an Acm group, a tBu group, and an S-tBu group, examples of the protective group of the indole group include a Boc group, and examples of the protective group of the imidazole group include a Boc group, a Bom group, a Bum group, and a Trt group.

The amino acid or peptide having an amino group protected by an amino protective group can be produced, for example, by reacting chloroformic acid 9-fluorenylmethyl ester with an amino acid or peptide having an amino group to be protected by an amino protective group in a solvent such as THF in the presence of a condensing agent.

The step b of the present invention is to condense the compound, and a reaction solvent used in the step b is a solvent containing an organic solvent. When the amino acid, peptide, or amino acid amide is protected by the carrier for liquid phase peptide synthesis used in the present invention, the amino acid, peptide, or amino acid amide protected by the carrier for liquid phase peptide synthesis is dissolved in an organic solvent, so that liquid phase peptide synthesis can be performed.

Examples of such an organic solvent include tetrahydrofuran (THF), dimethylformamide (DMF), cyclohexane, cyclopentyl methyl ether (CPME), methyl-tert-butyl ether (MTBE), 2-methyl THF, 4-methyl tetrahydropyran (4-methyl THP), isopropyl acetate, chloroform, dichloromethane, and N-methyl pyrrolidone, and THF, DMF, cyclohexane, CPME, MTBE, 2-methyl THF, 4-methyl THP, isopropyl acetate, and N-methyl pyrrolidone are preferable. A mixed solvent of two or more kinds of the above solvents may be used.

The condensation reaction can be performed by mixing a peptide bound to a carrier for liquid phase peptide synthesis, an amino group-protected amino acid, and a condensing agent in the solvent containing an organic solvent.

The amount of the amino group-protected amino acid used with respect to the peptide bound to a carrier for liquid phase peptide synthesis is usually 1.01 to 4 equivalents, preferably 1.03 to 3 equivalents, more preferably 1.05 to 2 equivalents, and further preferably 1.1 to 1.5 equivalents with respect to the peptide bound to a carrier for liquid phase peptide synthesis. In the peptide production method of the present invention, an active ester of an unreacted amino acid can be captured and inactivated by a scavenger to be added later. Therefore, even when an excess amount of amino group-protected amino acid is used, the problem of remaining of the amino group-protected amino acid does not occur.

As the condensing agent, those generally used in peptide synthesis can also be used in the present invention, and examples thereof include 4-(4,6-dimethoxy-1,3,5-triazine-2-yl)-4-methylmorphonium chloride (DMT-MM), O-(benzotriazole-1-yl)-1,1,3,3-tetramethyluronium hexafluorophosphate (HBTU), O-(7-azabenzotriazole-1-yl)-1,1,3,3-tetramethyluronium hexafluorophosphate (HATU), O-(6-chlorobenzotriazole-1-yl)-1,1,3,3-tetramethyluronium hexafluorophosphate (HBTU(6-Cl)), O-(benzotriazole-1-yl)-1,1,3,3-tetramethyluronium tetrafluoroborate (TBTU), O-(6-chlorobenzotriazole-1-yl)-1,1,3,3-tetramethyluronium tetrafluoroborate (TCTU), (1-cyano-2-ethoxy-2-oxoethylideneaminooxy)dimethylamino-morpholino-carbenium hexafluorophosphate (COMU), diisopropylcarbodiimide (DIPCI), dicyclohexylcarbodiimide (DCC), and 1-ethyl-3-(3-dimethylaminopropyl)carbodiimide hydrochloride (WSC). DMT-MM, HBTU, HATU, or COMU is preferable. The amount of the condensing agent used is preferably 1 to 4 equivalents, more preferably 1 to 2 equivalents, and further preferably 1.05 to 1.3 equivalents with respect to the peptide bound to a carrier for liquid phase peptide synthesis.

In the condensation step, an activating agent is preferably added in order to promote the reaction and suppress side reactions such as racemization. The activating agent described herein is a reagent that makes it easy to guide the amino acid to, for example, a corresponding active ester or a symmetric acid anhydride to form a peptide bond (amide bond) by coexistence with a condensing agent. As the activating agent, those generally used in peptide synthesis can be used. Examples thereof include 1-hydroxybenzotriazole (HOBt), ethyl 1-hydroxy-1H-1,2,3-triazole carboxylate (HOCt), 1-hydroxy-7-azabenzotriazole (HOAt), 3-hydroxy-4-ketobenzotriazine (HOOBt), N-hydroxysuccinimide (HOSu), N-hydroxyphthalimide (HOPht), N-hydroxy-5-norbornene-2,3-dicarboximide (HONb), pentafluorophenol, and ethyl cyano(hydroxyimino) acetate (Oxyma). HOBt, HOOBt, HOCt, HOAt, HONb, HOSu, and Oxyma are preferable. The amount of the activating agent used is preferably 1 to 4 equivalents, more preferably 1 to 2 equivalents, and further preferably 1.05 to 1.3 equivalents with respect to the peptide bound to a carrier for liquid phase peptide synthesis.

The amount of the solvent used is an amount at which the concentration at which, for example, the peptide bound to a carrier for liquid phase peptide synthesis is dissolved is preferably 0.1 mM to 1 M, more preferably 1 mM to 0.5 M.

The reaction temperature is, for example, preferably -20 to 40°C, more preferably 0 to 30°C, which is a temperature generally used in peptide synthesis. The reaction time (time for 1 cycle) is usually 0.5 to 30 hours.

The step c is to add an amino acid active ester scavenger to the reaction solution after the condensation reaction.

From the viewpoint of continuing the reaction in an organic solvent, the amino acid active ester scavenger is preferably an amino group-containing compound and particularly preferably an amino group-containing compound selected from the group consisting of divalent or higher water-soluble amines, alkylamines, aromatic amines, hydroxylamines, aminosulfonic acids, amino sulfates, aminophosphonic acids, aminophosphoric acids, and amino alcohols.

Examples of the divalent or higher water-soluble amines include 1-methylpiperazine, 4-aminopiperidine, diethylenetriamine, triaminoethylamine, 1-ethylpiperazine, N,N-dimethylethylenediamine, ethylenediamine, and piperazine, 1-methylpiperazine, 4-aminopiperidine, diethylenetriamine, N,N-dimethylethylenediamine, and ethylenediamine are preferable, 1-methylpiperazine, 4-aminopiperidine, N,N-dimethylethylenediamine, and diethylenetriamine are more preferable, and 1-methylpiperazine is further preferable.

Examples of the alkylamine that can be used include an alkylamine having 1 to 14 carbon atoms, an alkylamine having 2 to 10 carbon atoms is preferable, an alkylamine having 2 to 8 carbon atoms is more preferable, and an alkylamine having 3 to 4 carbon atoms is further preferable. Examples of the aromatic amine that can be used in the present invention include an aromatic amine having 6 to 14 carbon atoms, and an aromatic amine having 6 to 10 carbon atoms is preferable. Specific examples of the alkylamine, the aromatic amine, and the hydroxylamine include, but are not limited to, propylamine, butylamine, hexylamine, aniline, toluidine, 2,4,6-trimethylaniline, anisidine, phenetidine, and hydroxylamine, and propylamine is particularly preferable.

The aminosulfonic acids, amino sulfates, aminophosphonic acids, aminophosphoric acids, and amino alcohols are preferably compounds of Formulas (15) to (17) below.

That is, preferred are aminosulfonic acids and amino sulfates of the following Formula (15):

H₂N-R⁹-X⁷-SO₃H (15)

wherein R⁹¹ represents a divalent organic group having 1 to 10 carbon atoms, and X⁷ represents a single bond or an oxygen atom;
aminophosphonic acids and aminophosphonic acids of Formula (16):

   H₂N-R⁹²-X⁸-(P=O)(OH)₂ (16)
wherein R⁹² represents a divalent organic group having 1 to 10 carbon atoms, and X⁸ represents a single bond or an oxygen atom;
and amino alcohols of Formula (17):

   H₂N-CR⁹³R⁹⁴(CH₂OCH₂)n-CH₂OH (17)
wherein n represents an integer of 0 to 20, and R⁹³ and R⁹⁴ each independently represent a hydrogen atom, a methyl group, an ethyl group, or a hydroxymethyl group.

R⁹¹ in Formula (15) and R⁹² in Formula (16) are independently a divalent organic group having 1 to 10 carbon atoms, and preferable examples thereof include a linear or branched alkylene group having 1 to 10 carbon atoms and an arylene group having 6 to 10 carbon atoms. Specific examples thereof include a methylene group, an ethylene group, a trimethylene group, a propylene group, a tetramethylene group, a tetramethylene group, a pentamethylene group, a phenylene group, and a naphthylene group.

Among these, from the viewpoint of solubility of these compounds, a linear or branched alkylene group having 1 to 6 carbon atoms and an arylene group having 6 to 8 carbon atoms are more preferable, a linear or branched alkylene group having 1 to 6 carbon atoms is further preferable, and a linear or branched alkylene group having 1 to 5 carbon atoms is still more preferable.

In Formula (15), when X⁷ is a single bond, the compound is an aminosulfonic acid, and when X⁷ is an oxygen atom, the compound is an amino sulfate.

In Formula (16), when X⁸ is a single bond, the compound is an aminophosphonic acid, and when X⁸ is an oxygen atom, the compound is an aminophosphonic acid.

n in Formula (17) represents an integer of 0 to 20. In particular, n is preferably 0 to 20, more preferably 0 to 6, and further preferably 0 to 4. R⁹³ and R⁹⁴ in Formula (17) are preferably a hydrogen atom or a hydroxyl methyl group.

Among the amino group-containing compounds, it is more preferable to use a compound selected from the group consisting of aminosulfonic acids, amino sulfates, aminophosphonic acids, aminophosphoric acids, and amino alcohols, as the amino acid active ester scavenger. By using these compounds as a scavenger, not only the amino acid active ester can be eliminated, but also by-products such as dibenzofulbene (DBF) can be eliminated without acidic conditions, so that liquid phase production of the peptide can be performed in one pot without an isolation operation.

The amount of the amino group-containing compound added in the step c is preferably 1 to 10 equivalents, more preferably 1 to 6 equivalents, and further preferably 1 to 4 equivalents with respect to 1 equivalent of the active amino acid ester remaining theoretically. When the amount of the amino group-containing compound added is too small, scavenging (capturing) of the amino acid active ester becomes insufficient, and double hit in which the remaining amino acid active ester reacts with the amino group generated in the step d occurs to reduce purity and yield. On the other hand, when the amount of the amino group-containing compound added is too large, amino protective group deprotection reaction simultaneously proceeds, and double hit in which the remaining amino acid active ester reacts with the regenerated amino group occurs to reduce purity and yield.

The step d is to deprotect the amino protective group of the compound having an amino group protected by an amino protective group in the reaction solution.

The deprotection step of the amino protective group varies depending on the type of the amino protective group. For example, when the amino protective group is an Fmoc group, the reaction solution may be set to basic conditions. When the amino protective group is a Boc group, the reaction solution may be set to acidic conditions. When the amino protective group is a Cbz group, catalytic reduction may be performed. When the amino protective group is an Ac group, the amino protective group may be deprotected under strong acid or strong base conditions. Among them, the amino protective group is more preferably an Fmoc group for one-pot liquid phase synthesis.

The deproteciton step of the amino protective group when the amino protective group is an Fmoc group will be described.

The Fmoc deprotection step is only required to make the reaction solution basic, and an amine compound, for example, tertiary amines such as 1,8-diazabicyclo[5.4.0]-7-undecene (DBU), 1,5-diazabicyclo[4.3.0]-5-nonene (DBN), 1,4-diazabicyclo[2.2.2]-octane (DABCO), triethylamine, and tributylamine; divalent or higher water-soluble amines having at least one primary or secondary amino group such as 1-methylpiperazine, 4-aminopiperidine, diethylenetriamine, triaminoethylamine, 1-ethylpiperazine, N,N-dimethylethylenediamine, ethylenediamine, piperidine, and piperazine can be used. DBU, piperidine, 1-methylpiperazine, 4-aminopiperidine, and diethylenetriamine are preferable, DBU, piperidine, and 1-methylpiperazine are more preferable, and DBU is further preferable. In the present specification, the base used in the step c may be referred to as an amine compound.

The equivalent of the amine compound added in the step c is 1 to 30 equivalents, preferably 4 to 20 equivalents, and more preferably 4 to 10 equivalents with respect to the amount of the Fmoc group present in the system.

In addition to the amine compound, it is preferable to add a trapping agent of DBF (dibenzofulbene) generated by Fmoc deprotection reaction. Examples of the trapping agent of DBF used herein include a mercapto compound. Examples of the mercapto compound that can be used include an acid having a mercapto group, for example, a mercapto fatty acid or an alkali metal salt thereof, and a compound of Formula (18) or (19) below:

HS-L1-SO₃M (18)

HS-L2-PO₃M₂ (19)

wherein L1 and L2 each represent a divalent organic group, and M represents a hydrogen atom or an alkali metal.

The mercapto fatty acid or the alkali metal salt thereof is preferably mercapto C1-20 fatty acid or an alkali metal salt thereof and more preferably mercapto C1-6 fatty acid or an alkali metal salt thereof.

In Formula (18) or (19), L1 and L2 each represent a divalent organic group. The divalent organic group is preferably a divalent organic group having 1 to 10 carbon atoms, and more preferably a linear or branched alkylene group having 1 to 10 carbon atoms which may have a mercapto group, an arylene group having 6 to 10 carbon atoms which may have a mercapto group, and a heteroarylene group having 4 to 9 carbon atoms which may have a mercapto group. Specific examples thereof include a methylene group, an ethylene group, a trimethylene group, a propylene group, a mercaptotrimethylene group, a mercaptopropylene group, a tetramethylene group, a butylene group, a pentamethylene group, a phenylene group, a naphthylene group, an indole group, a benzimidazole group, a quinolyl group, and an isoquinoline group.

M represents a hydrogen atom or an alkali metal. Specific examples thereof include a hydrogen atom, sodium, and potassium.

Specific examples thereof include sodium mercaptomethane sulfonate, sodium 2-mercaptoethane sulfonate, 2-mercaptoethane sulfonic acid, sodium 3-mercaptopropane sulfonate, 1,3-dimercaptopropane sulfonic acid, sodium 2-mercaptobenzimidazole-5-sulfonate, sodium mercaptomethane phosphonate, mercaptoethane phosphonic acid, sodium 3-mercaptopropane phosphonate, and sodium 1,3-dimercaptopropane phosphonate.

The amount of the mercapto compound added is preferably 1 to 30 equivalents, more preferably 1 to 10 equivalents, and further preferably 1 to 5 equivalents with respect to the amount of DBF by-produced theoretically.

The amine compound and the mercapto compound may be added simultaneously, or the mercapto compound and the amine compound may be added in this order, or the amine compound may be added to deprotect the Fmoc group and then the mercapto compound may be added.

The Fmoc deprotection step may be performed at a temperature of -20 to 40°C for 5 minutes to 5 hours.

The step e is to add an aqueous solution to the reaction solution and perform liquid separation to obtain an organic solvent layer containing a condensate of the amino acid, peptide, or amino acid amide to which the carrier for liquid phase peptide synthesis is bound and the amino acid or peptide from which the amino protective group is deprotected.

After the step d of adding an aqueous solution to the reaction solution, the aqueous layer and the organic solvent layer are separated.

The aqueous layer contains a condensate of the amino acid or peptide from which the amino protective group is deprotected and an active ester scavenger, and a DBF-trapping agent adduct. That is, the DBF-trapping agent adduct by-produced in the deprotection step of the amino protective group is easily extracted into the aqueous layer only by the addition of the aqueous solution in the step e.

Meanwhile, the organic solvent layer contains a condensate of the amino acid, peptide, or amino acid amide to which the carrier for liquid phase peptide synthesis is bound and the amino acid or peptide from which the amino protective group is deprotected.

Examples of the aqueous solution to be used include water, a sodium chloride aqueous solution, a sodium carbonate aqueous solution, a potassium carbonate aqueous solution, a disodium hydrogen phosphate aqueous solution, a trisodium phosphate aqueous solution, a sodium hydrogen carbonate aqueous solution, a potassium hydrogen carbonate aqueous solution, a dipotassium hydrogen phosphate aqueous solution, and a tripotassium phosphate aqueous solution.

As described above, according to the step e of the present invention, it is not necessary to use an acidic aqueous solution simply by adding an aqueous solution and liquid separation, so that liquid separation failure between the amino acid active ester and the peptide as a product does not occur. Since solid-liquid separation is not required, one-pot synthesis is possible without isolating the liquid phase production of the peptide.

The obtained organic solvent layer can be further used for the condensation reaction with an arbitrary amino acid.

Examples of a sample to be analyzed of the present invention include a reaction solution, a concentrate thereof, or an extract thereof in an arbitrary step of the liquid phase peptide production method using a compound in which a carrier for liquid phase peptide synthesis and an amino acid, peptide, or amino acid amide are bound, that is, in an arbitrary step of the above steps a to e.

Examples of the sample containing a target peptide or final target peptide obtained by the method include the reaction solution of the step e, an extraction liquid thereof, a reaction solution obtained after all protective groups after liquid separation and the step e are deprotected, an extraction liquid thereof, and a solid or filtrate obtained by liquid separation and solid-liquid separation.

An analysis means is HPLC or supercritical liquid chromatography, and in the present invention, an alcohol is used instead of acetonitrile as an eluent for these kinds of liquid chromatography. When an alcohol is used, not only various peptides but also, for example, a carrier for liquid phase peptide synthesis and a peptide bound to a carrier for liquid phase peptide synthesis are dissolved, and these components can be simultaneously analyzed.

Examples of the alcohol to be used include alcohols having 1 to 4 carbon atoms such as ethanol, isopropanol, and n-butanol, ethanol and isopropanol are more preferable, and isopropanol is further preferable. The eluent may be a solution containing these alcohols, and may contain, for example, various organic solvents, acids, bases, salts, and counter ion forming products, in addition to the alcohols.

In the analysis method of the present invention, the operation itself may be performed according to an ordinary HPLC of supercritical liquid chromatography operation.

HPLC is preferably reversed-phase HPLC, and the stationary phase (column packing material) can be appropriately selected from those usually used in consideration of, for example, the length of the target peptide contained in the sample to be analyzed and the physical properties of the carrier for liquid phase peptide synthesis. Suitable examples thereof include octadecyl silica (ODS), and for example, the particle shape of the packing material, the pore size, and the density of ODS can also be appropriately selected. Examples of a commercially available column having the ODS as a stationary phase include MonoBis Column low-pressure ODS end cap (mesopore 11 nm) 3.2 x 150 mm (Kyoto Monotech Co., Ltd.), and Kinetex EVO C18 4.6 x 150 mm particle size of 2.6 um (manufactured by Phenomenex Inc.).

As a column used for supercritical fluid chromatography, a commercially available product such as BEH C18 1.7 um 2.1 x 50 mm 130 Å (Waters) can be used.

According to the present invention, it is possible to solve the problem in that the peptide bound to a carrier for liquid phase peptide synthesis is not dissolved in the eluent and the problem in that the peptide bound to a carrier for liquid phase peptide synthesis is not eluted. By adding an acidic compound to the eluent, the reaction rate of each step can be grasped by analysis using exactly the same eluent in a series of peptide synthesis. Since the analysis of the peptide component can be simultaneously performed, it is possible to determine whether or not the component derived from the carrier for liquid phase peptide synthesis is mixed in the target peptide (final target peptide) after deprotection, and the mixing ratio when the component is mixed in the target peptide.

### Examples

The present invention will be described in more detail with reference to Examples; however, the present invention is not limited to these Examples at all.

As the carrier for liquid phase peptide synthesis, the following compounds were used.
· TIPS2-OH(C11) type benzyl compound (manufactured by SEKISUI MEDICAL CO., LTD.) (sometimes referred to as "B-STag" hereinafter) TIPS represents a triisopropylsilyl group. 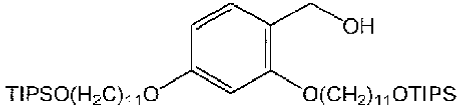
· TIPS2-OH(C11) type diphenylmethane compound (manufactured by SEKISUI MEDICAL CO., LTD.) (sometimes referred to as "D-STag" hereinafter) TIPS represents a triisopropylsilyl group.

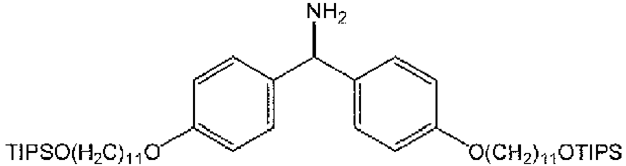

Fmoc-NH-(D-STag) is a compound in which an amino group of D-STag is protected with an Fmoc group.
· TIPS2-OH(C11) type xanthene compound (manufactured by SEKISUI MEDICAL CO., LTD.) (sometimes referred to as "X-STag" hereinafter) TIPS represents a triisopropylsilyl group.

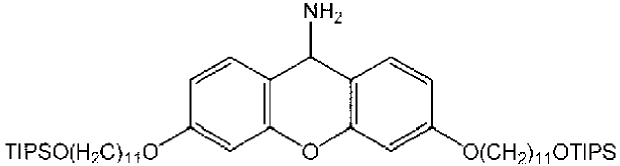

Fmoc-NH-(X-STag) is a compound in which an amino group of X-STag is protected with an Fmoc group.

In the following Examples, a compound in which B-STag is bound to Fmoc-protected tBu-protected glutamic acid (Fmoc-Glu(OtBu)-OH) is referred to as "Fmoc-Glu(OtBu)-O-(B-STag)", and the compound shows the following structure. It applies to not only when B-Stag is bound to Fmoc-Glu(OtBu)-OH, but also when B-STag is bound to another amino acid.

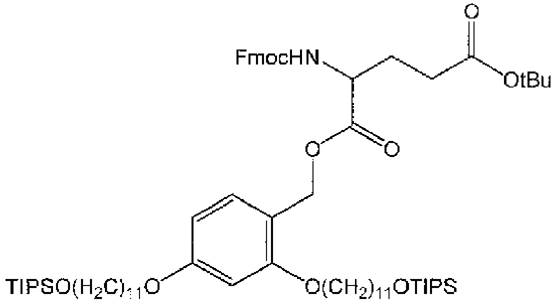

A compound in which D-STag is bound to Fmoc-protected tBu-protected threonine (Fmoc-Thr(tBu)-OH)) is referred to as "Fmoc-Thr(tBu)-NH-(D-STag)", and the compound shows the following structure.

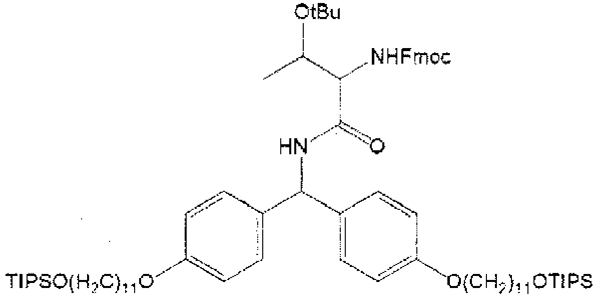

A compound in which X-STag is bound to Fmoc-protected tBu-protected threonine (Fmoc-Thr(tBu)-OH)) is referred to as "Fmoc-Thr(tBu)-NH-(X-STag)", and the compound shows the following structure.

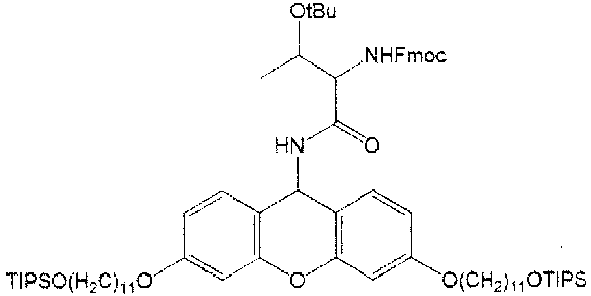

Analysis Condition 1 (HPLC)
Column: MonoBis Column low-pressure ODS end cap (mesopore 11 nm) 3.2 x 150 mm (Kyoto Monotech Co., Ltd.)
Mobile phase A: 0.1% formic acid-containing 5% isopropanol aqueous solution
Mobile phase B: 0.1% formic acid-containing 95% isopropanol aqueous solution
Flow rate: 1.0 mL/min
Column temperature: 60°C
Detection wavelength: 220 nm
Gradient conditions: 5% B (0 min) → 5% B (2 min) → 95% B (12 min) → 95% B (17.5 min) → 5% B (18 min) → 5% B (24 min)
Analysis Condition 2 (SFC)
Column: BEH C18 1.7 um 2.1 x 50 mm 130 Å (Waters)
Mobile phase A: CO2
Mobile phase B: Methanol/isopropanol/28% ammonia aqueous solution (500/500/1) solution
Pressure (ABPR): 2000 psi
Flow rate: 1.1 mL/min
Column temperature: 45°C
Detection wavelength: 220 nm
Gradient conditions: 5% B (0 min) → 15% B (5 min) → 25% B (6 min) → 25% B (7 min) → 5% B (8 min) → 5% B (10 min)
Analysis Condition 3 (HPLC)
Column: Kinetex EVO C18 4.6 x 150 mm particle size 2.6 um (manufactured by Phenomenex Inc.)
Mobile phase A: 0.025% TFA-containing aqueous solution
Mobile phase B: 0.025% TFA-containing acetonitrile solution
Flow rate: 1.0 mL/min
Column temperature: 60°C
Detection wavelength: 220 nm
Gradient conditions: 5% B (0 min) → 5% B (2 min) → 99% B (12 min) → 99% B (65 min) → 5% B (66 min) → 5% B (80 min)

### Example 1

### Synthesis of H-Asp-Ala-Asn-Cys-Glu-OH

### 1) Synthesis of H-Glu(OtBu)-O-(B-STag)

2.40 g (3.0 mmol) of B-STag was dissolved in 6 mL of CPME and 9 mL of THF, 3.21 g (7.5 mmol) of Fmoc-Glu(OtBu)-OH, 1.44 g (7.5 mmol) of WSCI·HCl, and 36.8 mg (0.3 mmol) of 4-dimethylaminopyridine were added, and the mixture was stirred at room temperature for 2 hours. After it was confirmed that B-STag was 5% or less with respect to Fmoc-Glu(OtBu)-O-(B-STag) as a product in Analysis Condition 1, 0.94 g (7.5 mmol) of taurine and 38 mL of DMSO were added, and the mixture was stirred at room temperature for 30 minutes. The mixture was cooled to 0°C, 1.42 g (8.6 mmol) of sodium 2-mercapto-1-ethanesulfonate was added, 5.1 mL (34 mmol) of DBU was added, and the mixture was stirred for 20 minutes. After it was confirmed that Fmoc-Glu(OtBu)-O-(B-STag) was 5% or less with respect to H-Glu(OtBu)-O-(B-STag) as a product in Analysis Condition 1, 75 mL of a 5% sodium carbonate aqueous solution was added dropwise, the temperature was raised to room temperature, and liquid separation was performed. To the obtained organic layer, 17 mL of 20% saline, 6 mL of a 5% sodium carbonate aqueous solution, and 1.2 mL of DMF were added, and liquid separation was performed. The organic layer was concentrated, and then 27 mL of CPME was added to the residue to obtain a CPME solution containing H-Glu(OtBu)-O-(B-STag).
Retention time (Analysis Condition 1): B-STag: 13.8 minutes; Fmoc-Glu(OtBu)-O-(B-STag): 14.1 minutes; H-Glu(OtBu)-O-(B-STag): 12.2 minutes

### 2) Synthesis of H-Cys(Trt)-Glu(OtBu)-O-(B-STag)

To the CPME solution containing H-Glu(OtBu)-O-(B-STag), 7 mL of DMF, 2.38 g (4.1 mmol) of Fmoc-Cys(Trt)-OH, 1.67 g (3.9 mmol) of COMU, and 2.1 mL of DIEPA were added, and the mixture was stirred at room temperature for 2 hours. After it was confirmed that H-Glu(OtBu)-O-(B-STag) was 5% or less with respect to Fmoc-Cys(Trt)-Glu(OtBu)-O-(B-STag) as a product in Analysis Condition 1, 0.55 g (3.9 mmol) of taurine and 16 mL of DMSO were added, and the mixture was stirred at room temperature for 2 hours. The mixture was cooled to 0°C, 1.19 g (3.9 mmol) of sodium 2-mercapto-1-ethanesulfonate was added, 2.7 mL (18 mmol) of DBU was added, and the mixture was stirred for 20 minutes. After it was confirmed that Fmoc-Cys(Trt)-Glu(OtBu)-O-(B-STag) was 5% or less with respect to H-Cys(Trt)-Glu(OtBu)-O-(B-STag) as a product in Analysis Condition 1, 9.6 mL of a 1 M sulfuric acid aqueous solution was added dropwise, 60 mL of a 5% sodium carbonate aqueous solution was then added, the temperature was raised to room temperature, and liquid separation was performed. To the obtained organic layer, 38 mL of 20% saline, 13 mL of a 5% sodium carbonate aqueous solution, and 2.7 mL of DMF were added, and liquid separation was performed. A CPME solution containing H-Cys(Trt)-Glu(OtBu)-O-(B-STag) was obtained.
Retention time (Analysis Condition 1): Fmoc-Cys(Trt)-Glu(OtBu)-O-(B-STag): 14.6 minutes; H-Cys(Trt)-Glu(OtBu)-O-(B-STag): 13.1 minutes

### 3) Synthesis of H-Asn(Trt)-Cys(Trt)-Glu(OtBu)-O-(B-STag)

To the CPME solution containing H-Cys(Trt)-Glu(OtBu)-O-(B-STag), 2 mL of CPME, 7 mL of DMF, 2.43 g (4.1 mmol) of Fmoc-Asn(Trt)-OH, 1.67 g (3.9 mmol) of COMU, and 2.1 mL of DIEPA were added, and the mixture was stirred at room temperature for 2 hours. After it was confirmed that H-Cys(Trt)-Glu(OtBu)-O-(B-STag) was 5% or less with respect to Fmoc-Asn(Trt)-Cys(Trt)-Glu(OtBu)-O-(B-STag) as a product in Analysis Condition 1, 0.55 g (3.9 mmol) of taurine and 16 mL of DMSO were added, and the mixture was stirred at room temperature for 40 minutes. The mixture was cooled to 0°C, 1.19 g (3.9 mmol) of sodium 2-mercapto-1-ethanesulfonate was added, 2.7 mL (18 mmol) of DBU was added, and the mixture was stirred for 1 hour. After it was confirmed that Fmoc-Asn(Trt)-Cys(Trt)-Glu(OtBu)-O-(B-STag) was 5% or less with respect to H-Asn(Trt)-Cys(Trt)-Glu(OtBu)-O-(B-STag) as a product in Analysis Condition 1, 60 mL of a 5% sodium carbonate aqueous solution was added dropwise, the temperature was raised to room temperature, and liquid separation was performed. To the obtained organic layer, 38 mL of 20% saline, 13 mL of a 5% sodium carbonate aqueous solution, and 2.7 mL of DMF were added, and liquid separation was performed. A CPME solution containing H-Asn(Trt)-Cys(Trt)-Glu(OtBu)-O-(B-STag) was obtained.
Retention time (Analysis Condition 1): Fmoc-Asn(Trt)-Cys(Trt)-Glu(OtBu)-O-(B-STag): 14.7 minutes; H-Asn(Trt)-Cys(Trt)-Glu(OtBu)-O-(B-STag): 13.0 minutes

### 4) Synthesis of H-Ala-Asn(Trt)-Cys(Trt)-Glu(OtBu)-O-(B-STag)

To the CPME solution containing H-Asn(Trt)-Cys(Trt)-Glu(OtBu)-O-(B-STag), 2 mL of CPME, 7 mL of DMF, 1.33 g (4.1 mmol) of Fmoc-Ala-OH monohydrate, 1.67 g (3.9 mmol) of COMU, and 2.1 mL of DIEPA were added, and the mixture was stirred at room temperature for 1.5 hours. After it was confirmed that H-Asn(Trt)-Cys(Trt)-Glu(OtBu)-O-(B-STag) was 5% or less with respect to Fmoc-Ala-Asn(Trt)-Cys(Trt)-Glu(OtBu)-O-(B-STag) as a product in Analysis Condition 1, 0.55 g (3.9 mmol) of taurine and 16 mL of DMSO were added, and the mixture was stirred at room temperature for 30 minutes. The mixture was cooled to 0°C, 1.19 g (3.9 mmol) of sodium 2-mercapto-1-ethanesulfonate and 1.3 mL of DMF were added, 2.7 mL (18 mmol) of DBU was added, and the mixture was stirred for 1 hour. After it was confirmed that Fmoc-Ala-Asn(Trt)-Cys(Trt)-Glu(OtBu)-O-(B-STag) was 5% or less with respect to H-Ala-Asn(Trt)-Cys(Trt)-Glu(OtBu)-O-(B-STag) as a product in Analysis Condition 1, 9.6 mL of a 1 M sulfuric acid aqueous solution was added dropwise, 60 mL of a 5% sodium carbonate aqueous solution was then added, the temperature was raised to room temperature, and liquid separation was performed. To the obtained organic layer, 38 mL of 20% saline, 13 mL of a 5% sodium carbonate aqueous solution, and 2.7 mL of DMF were added, and liquid separation was performed. A CPME solution containing H-Ala-Asn(Trt)-Cys(Trt)-Glu(OtBu)-O-(B-STag) was obtained.
Retention time (Analysis Condition 1): Fmoc-Ala-Asn(Trt)-Cys(Trt)-Glu(OtBu)-O-(B-STag): 14.5 minutes; H-Ala-Asn(Trt)-Cys(Trt)-Glu(OtBu)-O-(B-STag): 12.7 minutes

### 5) Synthesis of H-Asp(OtBu)-Ala-Asn(Trt)-Cys(Trt)-Glu(OtBu)-O-(B-STag)

To the CPME solution containing H-Ala-Asn(Trt)-Cys(Trt)-Glu(OtBu)-O-(B-STag), 2 mL of CPME, 7 mL of DMF, 1.67 g (4.1 mmol) of Fmoc-Asp(OtBu)-OH, 1.67 g (3.9 mmol) of COMU, and 2.1 mL of DIEPA were added, and the mixture was stirred at room temperature for 1 hour. After it was confirmed that H-Ala-Asn(Trt)-Cys(Trt)-Glu(OtBu)-O-(B-STag) was 5% or less with respect to Fmoc-Asp(OtBu)-Ala-Asn(Trt)-Cys(Trt)-Glu(OtBu)-O-(B-STag) as a product in Analysis Condition 1, 0.55 g (3.9 mmol) of taurine and 16 mL of DMSO were added, and the mixture was stirred at room temperature for 30 minutes. The mixture was cooled to 0°C, 1.19 g (3.9 mmol) of sodium 2-mercapto-1-ethanesulfonate and 1.3 mL of DMF were added, 2.7 mL (18 mmol) of DBU was added, and the mixture was stirred for 40 minutes. After it was confirmed that Fmoc-Asp(OtBu)-Ala-Asn(Trt)-Cys(Trt)-Glu(OtBu)-O-(B-STag) was 5% or less with respect to H-Asp(OtBu)-Ala-Asn(Trt)-Cys(Trt)-Glu(OtBu)-O-(B-STag) as a product in Analysis Condition 1, 9.6 mL of a 1 M sulfuric acid aqueous solution was added dropwise, 60 mL of a 5% sodium carbonate aqueous solution was then added, the temperature was raised to room temperature, and liquid separation was performed. To the obtained organic layer, 38 mL of 20% saline, 13 mL of a 5% sodium carbonate aqueous solution, and 2.7 mL of DMF were added, and liquid separation was performed. A CPME solution containing H-Asp(OtBu)-Ala-Asn(Trt)-Cys(Trt)-Glu(OtBu)-O-(B-STag) was obtained.

The obtained CPME solution was concentrated under reduced pressure, 40 mL of MeCN was added to the residue, the precipitated solid was collected by filtration, and the obtained solid was dried under reduced pressure at 30°C. 4.58 g of H-Asp(OtBu)-Ala-Asn(Trt)-Cys(Trt)-Glu(OtBu)-O-(B-STag) was obtained.
Retention time (Analysis Condition 1): Fmoc-Asp(OtBu)-Ala-Asn(Trt)-Cys(Trt)-Glu(OtBu)-O-(B-STag): 14.5 minutes; H-Asp(OtBu)-Ala-Asn(Trt)-Cys(Trt)-Glu(OtBu)-O-(B-STag): 13.1 minutes

### 6) Synthesis of H-Asp-Ala-Asn-Cys-Glu-OH

To 962 mg (0.50 mmol) of H-Asp(OtBu)-Ala-Asn(Trt)-Cys(Trt)-Glu(OtBu)-O-(B-STag), 9.5 mL of trifluoroacetic acid, 0.29 mL of water, 0.29 mL of triisopropylsilane, 865 mg of dithiothreitol, and 0.58 mL of anisole were added, and the mixture was stirred at room temperature for 2 hours. The reaction solution was cooled to 0°C, 72 mL of diisopropyl ether was slowly added dropwise, and a precipitate was collected by filtration. The precipitate collected by filtration was washed with 10 mL of diisopropyl ether three times, and then the precipitate was dried under reduced pressure to obtain 275 mg of H-Asp-Ala-Asn-Cys-Glu-OH. As a result of analyzing the obtained H-Asp-Ala-Asn-Cys-Glu-OH in Analysis Condition 1, the purity was 80.1%, and B-STag and the B-STag-derived compound produced by treating B-STag with trifluoroacetic acid were not detected.
Retention time (Analysis Condition 1): H-Asp-Ala-Asn-Cys-Glu-OH: 0.9 minutes

The purity was calculated as the ratio of the peak area of the target peptide to the total peak area over a retention time of 0 to 19 minutes. The same applies to the following Examples.

### Example 2

### Synthesis of H-Asp-Ala-Asn-Cys-Glu-OH

### 1) Synthesis of H-Glu(OtBu)-O-(B-STag)

0.79 g (1.0 mmol) of B-STag was dissolved in 2 mL of CPME and 3 mL of THF, 1.06 g (2.5 mmol) of Fmoc-Glu(OtBu)-OH, 0.48 g (2.5 mmol) of WSCI·HCl, and 12.2 mg (0.1 mmol) of 4-dimethylaminopyridine were added, and the mixture was stirred at room temperature for 2 hours. After it was confirmed that B-STag was 5% or less with respect to Fmoc-Glu(OtBu)-O-(B-STag) as a product in Analysis Condition 2, 150 µL (2.5 mmol) of 2-aminoethanol was added, and the mixture was stirred at room temperature for 2 hours. The mixture was cooled to 0°C, 0.74 g (4.5 mmol) of sodium 2-mercapto-1-ethanesulfonate and 4.5 mL of DMSO were added, 1.7 mL (11.3 mmol) of DBU was added, and the mixture was stirred for 1 hour. After it was confirmed that Fmoc-Glu(OtBu)-O-(B-STag) was 5% or less with respect to H-Glu(OtBu)-O-(B-STag) as a product in Analysis Condition 2, 6 mL of a 1 M sulfuric acid aqueous solution was added dropwise, 19 mL of a 5% sodium carbonate aqueous solution was added, the temperature was then raised to room temperature, and liquid separation was performed. To the obtained organic layer, 20 mL of 20% saline was added, and liquid separation was performed. The organic layer was concentrated, and then 9 mL of CPME was added to the residue to obtain a CPME solution containing H-Glu(OtBu)-O-(B-STag).
Retention time (Analysis Condition 2): B-STag: 2.1 minutes; Fmoc-Glu(OtBu)-O-(B-STag): 3.1 minutes; H-Glu(OtBu)-O-(B-STag): 3.8 minutes

### 2) Synthesis of H-Cys(Trt)-Glu(OtBu)-O-(B-STag)

To the CPME solution containing H-Glu(OtBu)-O-(B-STag), 2 mL of DMF, 0.79 g (1.4 mmol) of Fmoc-Cys(Trt)-OH, 0.57 g (1.3 mmol) of COMU, and 0.71 mL of DIEPA were added, and the mixture was stirred at room temperature for 1 hour. After it was confirmed that H-Glu(OtBu)-O-(B-STag) was 5% or less with respect to Fmoc-Cys(Trt)-Glu(OtBu)-O-(B-STag) as a product in Analysis Condition 2, 81 µL (1.4 mmol) of 2-aminoethanol was added, and the mixture was stirred at room temperature for 1.5 hours. The mixture was cooled to 0°C, 0.40 g (1.3 mmol) of sodium 2-mercapto-1-ethanesulfonate and 2.4 mL of DMSO were added, 0.91 mL (6.1 mmol) of DBU was added, and the mixture was stirred for 25 minutes. After it was confirmed that Fmoc-Cys(Trt)-Glu(OtBu)-O-(B-STag) was 5% or less with respect to H-Cys(Trt)-Glu(OtBu)-O-(B-STag) as a product in Analysis Condition 2, 3.2 mL of a 1 M sulfuric acid aqueous solution was added dropwise, 13 mL of a 5% sodium carbonate aqueous solution was then added, the temperature was raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Cys(Trt)-Glu(OtBu)-O-(B-STag) was obtained.
Retention time (Analysis Condition 2): Fmoc-Cys(Trt)-Glu(OtBu)-O-(B-STag): 4.4 minutes; H-Cys(Trt)-Glu(OtBu)-O-(B-STag): 5.0 minutes

### 3) Synthesis of H-Asn(Trt)-Cys(Trt)-Glu(OtBu)-O-(B-STag)

To the CPME solution containing H-Cys(Trt)-Glu(OtBu)-O-(B-STag), 0.5 mL of CPME, 2 mL of DMF, 0.81 g (1.4 mmol) of Fmoc-Asn(Trt)-OH, 0.56 g (1.3 mmol) of COMU, and 0.71 mL of DIEPA were added, and the mixture was stirred at room temperature for 55 minutes. After it was confirmed that H-Cys(Trt)-Glu(OtBu)-O-(B-STag) was 5% or less with respect to Fmoc-Asn(Trt)-Cys(Trt)-Glu(OtBu)-O-(B-STag) as a product in Analysis Condition 2, 81 µL (1.4 mmol) of 2-aminoethanol was added, and the mixture was stirred at room temperature for 25 minutes. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 75 minutes. After it was confirmed that Fmoc-Asn(Trt)-Cys(Trt)-Glu(OtBu)-O-(B-STag) was 5% or less with respect to H-Asn(Trt)-Cys(Trt)-Glu(OtBu)-O-(B-STag) as a product in Analysis Condition 2, 3.2 mL of a 1 M sulfuric acid aqueous solution was added dropwise, 13 mL of a 5% sodium carbonate aqueous solution was then added, the temperature was raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Asn(Trt)-Cys(Trt)-Glu(OtBu)-O-(B-STag) was obtained.
Retention time (Analysis Condition 2): Fmoc-Asn(Trt)-Cys(Trt)-Glu(OtBu)-O-(B-STag): 5.6 minutes; H-Asn(Trt)-Cys(Trt)-Glu(OtBu)-O-(B-STag): 6.6 minutes

### 4) Synthesis of H-Ala-Asn(Trt)-Cys(Trt)-Glu(OtBu)-O-(B-STag)

To the CPME solution containing H-Asn(Trt)-Cys(Trt)-Glu(OtBu)-O-(B-STag), 0.5 mL of CPME, 2 mL of DMF, 0.47 g (1.5 mmol) of Fmoc-Ala-OH monohydrate, 0.64 g (1.5 mmol) of COMU, and 0.78 mL (4.5 mmol) of DIEPA were added, and the mixture was stirred at room temperature for 1 hour. After it was confirmed that H-Asn(Trt)-Cys(Trt)-Glu(OtBu)-O-(B-STag) was 5% or less with respect to Fmoc-Ala-Asn(Trt)-Cys(Trt)-Glu(OtBu)-O-(B-STag) as a product in Analysis Condition 2, 89 µL (1.5 mmol) of 2-aminoethanol was added, and the mixture was stirred at room temperature for 2 hours. The mixture was cooled to 0°C, 0.40 g (2.5 mmol) of sodium 2-mercapto-1-ethanesulfonate and 2.5 mL of DMSO were added, 0.91 mL (6.1 mmol) of DBU was added, and the mixture was stirred for 1 hour. After it was confirmed that Fmoc-Ala-Asn(Trt)-Cys(Trt)-Glu(OtBu)-O-(B-STag) was 5% or less with respect to H-Ala-Asn(Trt)-Cys(Trt)-Glu(OtBu)-O-(B-STag) as a product in Analysis Condition 2, 3.2 mL of a 1 M sulfuric acid aqueous solution was added dropwise, 13 mL of a 5% sodium carbonate aqueous solution was then added, the temperature was raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Ala-Asn(Trt)-Cys(Trt)-Glu(OtBu)-O-(B-STag) was obtained.
Retention time (Analysis Condition 2): Fmoc-Ala-Asn(Trt)-Cys(Trt)-Glu(OtBu)-O-(B-STag): 6.1 minutes; H-Ala-Asn(Trt)-Cys(Trt)-Glu(OtBu)-O-(B-STag): 7.2 minutes

### 5) Synthesis of H-Asp(OtBu)-Ala-Asn(Trt)-Cys(Trt)-Glu(OtBu)-O-(B-STag)

To the CPME solution containing H-Ala-Asn(Trt)-Cys(Trt)-Glu(OtBu)-O-(B-STag), 0.5 mL of CPME, 2 mL of DMF, 0.56 g (1.4 mmol) of Fmoc-Asp(OtBu)-OH, 0.56 g (1.3 mmol) of COMU, and 0.71 mL (4.1 mmol) of DIEPA were added, and the mixture was stirred at room temperature for 1 hour. After it was confirmed that H-Ala-Asn(Trt)-Cys(Trt)-Glu(OtBu)-O-(B-STag) was 5% or less with respect to Fmoc-Asp(OtBu)-Ala-Asn(Trt)-Cys(Trt)-Glu(OtBu)-O-(B-STag) as a product in Analysis Condition 2, 81 µL (1.4 mmol) of 2-aminoethanol was added, and the mixture was stirred at room temperature for 70 minutes. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 75 minutes. After it was confirmed that Fmoc-Asp(OtBu)-Ala-Asn(Trt)-Cys(Trt)-Glu(OtBu)-O-(B-STag) was 5% or less with respect to H-Asp(OtBu)-Ala-Asn(Trt)-Cys(Trt)-Glu(OtBu)-O-(B-STag) as a product in Analysis Condition 2, 3.2 mL of water was added dropwise, 13 mL of a 5% sodium carbonate aqueous solution was then added, the temperature was raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Asp(OtBu)-Ala-Asn(Trt)-Cys(Trt)-Glu(OtBu)-O-(B-STag) was obtained.

The obtained CPME solution was concentrated under reduced pressure, 40 mL of MeCN was added to the residue, the precipitated solid was collected by filtration, and the obtained solid was dried under reduced pressure at 30°C. 1.56 g of H-Asp(OtBu)-Ala-Asn(Trt)-Cys(Trt)-Glu(OtBu)-O-(B-STag) was obtained.
Retention time (Analysis Condition 2): Fmoc-Asp(OtBu)-Ala-Asn(Trt)-Cys(Trt)-Glu(OtBu)-O-(B-STag): 6.3 minutes; H-Asp(OtBu)-Ala-Asn(Trt)-Cys(Trt)-Glu(OtBu)-O-(B-STag): 7.0 minutes

### 6) Synthesis of H-Asp-Ala-Asn-Cys-Glu-OH

To 962 mg (0.50 mmol) of H-Asp(OtBu)-Ala-Asn(Trt)-Cys(Trt)-Glu(OtBu)-O-(B-STag), 7.5 mL of trifluoroacetic acid, 0.21 mL of water, 0.21 mL of triisopropylsilane, and 0.42 mL of 3,6-dioxa-1,8-octanedithiol were added, and the mixture was stirred at room temperature for 2 hours. The reaction solution was cooled to 0°C, 60 mL of MTBE was slowly added dropwise, and a precipitate was collected by filtration. The precipitate collected by filtration was washed with 10 mL of MTBE three times, and then the precipitate was dried under reduced pressure to obtain 275 mg of H-Asp-Ala-Asn-Cys-Glu-OH. As a result of analyzing the obtained H-Asp-Ala-Asn-Cys-Glu-OH in Analysis Condition 1, the purity was 74.2%, B-STag was not detected, and 0.6% of the B-STag-derived compound produced by treating B-STag with trifluoroacetic acid was detected.

### Example 3

To 0.16 g (0.2 mmol) of B-STag, 3.2 mL of trifluoroacetic acid, 0.09 mL of water, 0.09 mL of triisopropylsilane, and 0.18 mL of 3,6-dioxa-1,8-octanedithiol were added, and the mixture was stirred at room temperature for 2 hours. The reaction solution was analyzed in Analysis Condition 1 and Analysis Condition 2 to detect the B-STag-derived compound.
Retention time (Analysis Condition 1): 11.2 minutes; 11.6 minutes; 12.3 minutes; 12.4 minutes; 12.6 minutes
Retention time (Analysis Condition 2): 0.2 minutes; 0.4 minutes; 0.6 minutes; 1.1 minutes; 2.1 minutes

In Example 2, it was found that since the target peptide was crystallized using MTBE and separated and purified after deprotection of, for example, B-STag, only a small amount of the B-STag-derived compound was detected; however, the B-STag-derived compound in the mixed liquid before the crystallization operation can be detected with good separation performance using any of Analysis Conditions 1 and 2.

### Example 4

### Synthesis of H-Ser-Met-Ile-Leu-Glu-OH

### 1) Synthesis of H-Glu(O-B-STag)-OtBu

1.59 g (2.0 mmol) of B-STag was dissolved in 4 mL of CPME and 6 mL of THF, 2.13 g (5.0 mmol) of Fmoc-Glu-OtBu, 0.96 g (5.0 mmol) of WSCI·HCl, and 24.5 mg (0.2 mmol) of 4-dimethylaminopyridine were added, and the mixture was stirred at room temperature for 5 hours. After it was confirmed that B-STag was 5% or less with respect to Fmoc-Glu(O-B-STag)-OtBu as a product in Analysis Condition 1, 300 µL (5.0 mmol) of 2-aminoethanol (AE) was added, and the mixture was stirred at room temperature for 1 hour. The mixture was cooled to 0°C, 1.48 g (9.0 mmol) of sodium 2-mercapto-1-ethanesulfonate and 9 mL of DMSO were added, 3.4 mL (22.5 mmol) of DBU was added, and the mixture was stirred for 20 minutes. After it was confirmed that Fmoc-Glu(O-B-STag)-OtBu was 5% or less with respect to H-Glu(O-B-STag)-OtBu as a product in Analysis Condition 1, 12 mL of a 1 M sulfuric acid aqueous solution was added dropwise, 38 mL of a 5% sodium carbonate aqueous solution was then added, the temperature was raised to room temperature, and liquid separation was performed. To the obtained organic layer, 10 mL of 20% saline, 4 mL of a 5% sodium carbonate aqueous solution, and 0.8 mL of DMF were added, and liquid separation was performed. The organic layer was concentrated under reduced pressure, and 6 mL of CPME was added to the residue to obtain 15.2 g of a CPME solution containing H-Glu(O-B-STag)-OtBu.
Retention time (Analysis Condition 1): B-STag: 13.8 minutes; Fmoc-Glu(O-B-STag)-OtBu: 14.3 minutes; H-Glu(O-B-STag)-OtBu: 12.3 minutes

### 2) Synthesis of H-Leu-Glu(O-B-STag)-OtBu

To 7.6 g (corresponding to 1 mmol) of the CPME solution containing H-Glu(O-B-STag)-OtBu, 1.9 mL of CMPE, 2 mL of DMF, 0.48 g (1.4 mmol) of Fmoc-Leu-OH, 0.56 g (1.3 mmol) of COMU, and 0.71 mL (4.1 mmol) of DIEPA were added, and the mixture was stirred at room temperature for 1 hour. After it was confirmed that H-Glu(O-B-STag)-OtBu was 5% or less with respect to Fmoc-Leu-Glu(O-B-STag)-OtBu as a product in Analysis Condition 1, 81 µL (1.4 mmol) of AE was added, and the mixture was stirred at room temperature for 90 minutes. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 20 minutes. After it was confirmed that Fmoc-Leu-Glu(O-B-STag)-OtBu was 5% or less with respect to H-Leu-Glu(O-B-STag)-OtBu as a product in Analysis Condition 1, 3.2 mL of a 1 M sulfuric acid aqueous solution was added dropwise, 13 mL of a 5% sodium carbonate aqueous solution was then added, the temperature was raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Leu-Glu(O-B-STag)-OtBu was obtained.
Retention time (Analysis Condition 1): Fmoc-Leu-Glu(O-B-STag)-OtBu: 14.2 minutes; H-Leu-Glu(O-B-STag)-OtBu: 12.4 minutes

### 3) Synthesis of H-Ile-Leu-Glu(O-B-STag)-OtBu

To the CPME solution containing H-Leu-Glu(O-B-STag)-OtBu, 1 mL of CPME, 2 mL of DMF, 0.48 g (1.4 mmol) of Fmoc-Ile-OH, 0.56 g (1.3 mmol) of COMU, and 0.71 mL (4.1 mmol) of DIEPA were added, and the mixture was stirred at room temperature for 55 minutes. After it was confirmed that H-Leu-Glu(O-B-STag)-OtBu was 5% or less with respect to Fmoc-Ile-Leu-Glu(O-B-STag)-OtBu as a product in Analysis Condition 1, 81 µL (1.4 mmol) of AE was added, and the mixture was stirred at room temperature for 25 minutes. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 75 minutes. After it was confirmed that Fmoc-Ile-Leu-Glu(O-B-STag)-OtBu was 5% or less with respect to H-Ile-Leu-Glu(O-B-STag)-OtBu as a product in Analysis Condition 1, 3.2 mL of a 1 M sulfuric acid aqueous solution was added dropwise, 13 mL of a 5% sodium carbonate aqueous solution was then added, the temperature was raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Ile-Leu-Glu(O-B-STag)-OtBu was obtained.
Retention time (Analysis Condition 1): Fmoc-Ile-Leu-Glu(O-B-STag)-OtBu: 14.3 minutes; H-Ile-Leu-Glu(O-B-STag)-OtBu: 12.4 minutes

### 4) Synthesis of H-Met-Ile-Leu-Glu(O-B-STag)-OtBu

To the CPME solution containing H-Ile-Leu-Glu(O-B-STag)-OtBu, 1 mL of CPME, 2 mL of DMF, 0.56 g (1.5 mmol) of Fmoc-Met-OH, 0.65 g (1.5 mmol) of COMU, and 0.78 mL (4.5 mmol) of DIEPA were added, and the mixture was stirred at room temperature for 50 minutes. After it was confirmed that H-Ile-Leu-Glu(O-B-STag)-OtBu was 5% or less with respect to Fmoc-Met-Ile-Leu-Glu(O-B-STag)-OtBu as a product in Analysis Condition 1, 89 µL (1.5 mmol) of AE was added, and the mixture was stirred at room temperature for 25 minutes. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 1 hour. After it was confirmed that Fmoc-Met-Ile-Leu-Glu(O-B-STag)-OtBu was 5% or less with respect to H-Met-Ile-Leu-Glu(O-B-STag)-OtBu as a product in Analysis Condition 1, 3.2 mL of a 1 M sulfuric acid aqueous solution was added dropwise, 13 mL of a 5% sodium carbonate aqueous solution was then added, the temperature was raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Met-Ile-Leu-Glu(O-B-STag)-OtBu was obtained.
Retention time (Analysis Condition 1): Fmoc-Met-Ile-Leu-Glu(O-B-STag)-OtBu: 14.1 minutes; H-Met-Ile-Leu-Glu(O-B-STag)-OtBu: 12.5 minutes

### 5) Synthesis of H-Ser(tBu)-Met-Ile-Leu-Glu(O-B-STag)-OtBu

To the CPME solution containing H-Met-Ile-Leu-Glu(O-B-STag)-OtBu, 1 mL of CPME, 2 mL of DMF, 0.52 g (1.4 mmol) of Fmoc-Ser(tBu)-OH, 0.56 g (1.4 mmol) of COMU, and 0.71 mL (4.1 mmol) of DIEPA were added, and the mixture was stirred at room temperature for 1 hour. After it was confirmed that H-Met-Ile-Leu-Glu(O-B-STag)-OtBu was 5% or less with respect to Fmoc-Ser(tBu)-Met-Ile-Leu-Glu(O-B-STag)-OtBu as a product in Analysis Condition 1, 81 µL (1.4 mmol) of AE was added, and the mixture was stirred at room temperature for 2 hours. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 75 minutes. After it was confirmed that Fmoc-Ser(tBu)-Met-Ile-Leu-Glu(O-B-STag)-OtBu was 5% or less with respect to H-Ser(tBu)-Met-Ile-Leu-Glu(O-B-STag)-OtBu as a product in Analysis Condition 1, 3.2 mL of a 1 M sulfuric acid aqueous solution was added dropwise, 13 mL of a 5% sodium carbonate aqueous solution was then added, the temperature was raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Ser(tBu)-Met-Ile-Leu-Glu(O-B-STag)-OtBu was obtained.

The obtained CPME solution was concentrated under reduced pressure to obtain 1.64 g of H-Ser(tBu)-Met-Ile-Leu-Glu(O-B-STag)-OtBu as a residue.
Retention time (Analysis Condition 1): Fmoc-Ser(tBu)-Met-Ile-Leu-Glu(O-B-STag)-OtBu: 14.1 minutes; H-Ser(tBu)-Met-Ile-Leu-Glu(O-B-STag)-OtBu: 12.6 minutes

### 6) Synthesis of H-Ser-Met-Ile-Leu-Glu-OH

To 1.64 g (corresponding to 1.0 mmol) of the H-Ser(tBu)-Met-Ile-Leu-Glu(O-B-STag)-OtBu, 14.3 mL of trifluoroacetic acid, 0.36 mL of water, 0.36 mL of triisopropylsilane, and 0.71 mL of 3,6-dioxa-1,8-octanedithiol were added, and the mixture was stirred at room temperature for 2 hours. The reaction solution was cooled to 0°C, 81 mL of MTBE was slowly added dropwise, and a precipitate was collected by filtration. The precipitate collected by filtration was washed with 14 mL of MTBE three times, and then the precipitate was dried under reduced pressure to obtain 523 mg of H-Ser-Met-Ile-Leu-Glu-OH. As a result of analyzing the obtained H-Ser-Met-Ile-Leu-Glu-OH in Analysis Condition 1, the purity was 62.3%, and B-STag and the B-STag-derived compound produced by treating B-STag with trifluoroacetic acid were not detected.

The HPLC analysis results in Analysis Condition 1 are shown in Fig. 1. A peak at 1.9 minutes indicates H-Ser-Met-Ile-Leu-Glu-OH as the target compound. The B-STag-derived compound detected in Example 3 (retention time: 11.2 minutes to 12.6 minutes) was not detected.
Retention time (Analysis Condition 1): H-Ser-Met-Ile-Leu-Glu-OH: 1.9 minutes

### Example 5

### Synthesis of H-Gln-Trp-Glu-Arg-Thr-NH₂

### 1) Synthesis of H-Thr(tBu)-NH-(D-STag)

After 1.09 g (1.0 mmol) of Fmoc-NH-(D-STag) was dissolved in 9 mL of CPME and 2 mL of DMF and cooled to 0°C, 0.32 mL (1.9 mmol) of DIEPA and 0.30 g (1.8 mmol) of sodium 2-mercapto-1-ethanesulfonate were added, and 0.67 mL (4.5 mmol) of DBU was added dropwise thereto. After the mixture was stirred at 0°C for 1 hour and it was confirmed that Fmoc-NH-(D-STag) was 5% or less with respect to NH₂-(D-STag) as a product in Analysis Condition 1, 2.4 mL of a 1 M sulfuric acid aqueous solution was added dropwise, 10 mL of a 5% sodium carbonate aqueous solution was added, the temperature was then raised to room temperature, and liquid separation was performed. To the obtained organic layer, 25 mL of 20% saline, 8 mL of a 5% sodium carbonate aqueous solution, and 1.8 mL of DMF were added, and liquid separation was performed to obtain a CPME solution containing NH₂-(D-STag).

To the CPME solution containing NH₂-(D-STag), 0.6 mL of CPME, 2 mL of DMF, 0.55 g (1.4 mmol) of Fmoc-Thr(tBu)-OH, 0.56 g (1.3 mmol) of COMU, and 0.71 mL (4.1 mmol) of DIEPA were added, and the mixture was stirred at room temperature for 70 minutes. After it was confirmed that NH₂-(D-STag) was 5% or less with respect to Fmoc-Thr(tBu)-NH-(D-STag) as a product in Analysis Condition 1, 62 µL (0.5 mmol) of 2-[2-(2-aminoethoxy)ethoxy]ethanol (AEEE) was added, and the mixture was stirred at room temperature for 30 minutes. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 70 minutes. After it was confirmed that Fmoc-Thr(tBu)-NH-(D-STag) was 5% or less with respect to H-Thr(tBu)-NH-(D-STag) as a product in Analysis Condition 1, 3.2 mL of a 1 M sulfuric acid aqueous solution was added dropwise, 13 mL of a 5% sodium carbonate aqueous solution was added, the temperature was then raised to room temperature, and liquid separation was performed. To the obtained organic layer, 25 mL of 20% saline, 8 mL of a 5% sodium carbonate aqueous solution, and 1.8 mL of DMF were added, and liquid separation was performed to obtain a CPME solution containing H-Thr(tBu)-NH-(D-STag).
Retention time (Analysis Condition 1): Fmoc-NH-(D-STag): 14,5 minutes; NH₂-(D-STag): 12.0 minutes; Fmoc-Thr(tBu)-NH-(D-STag): 14.5 minutes; H-Thr(tBu)-NH-(D-STag): 12.4 minutes

### 2) Synthesis of H-Arg(Pbf)-Thr(tBu)-NH-(D-STag)

To the CPME solution containing H-Thr(tBu)-NH-(D-STag), 0.7 mL of CPME, 2 mL of DMF, 0.88 g (1.4 mmol) of Fmoc-Arg(Pbf)-OH, 0.56 g (1.3 mmol) of COMU, and 0.71 mL (4.1 mmol) of DIEPA were added, and the mixture was stirred at room temperature for 1 hour. After it was confirmed that H-Thr(tBu)-NH-(D-STag) was 5% or less with respect to Fmoc-Arg(Pbf)-Thr(tBu)-NH-(D-STag) as a product in Analysis Condition 1, 62 µL (0.5 mmol) of AEEE was added, and the mixture was stirred at room temperature for 30 minutes. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 50 minutes. After it was confirmed that Fmoc-Arg(Pbf)-Thr(tBu)-NH-(D-STag) was 5% or less with respect to H-Arg(Pbf)-Thr(tBu)-NH-(D-STag) as a product in Analysis Condition 1, 13 mL of a 5% sodium carbonate aqueous solution was added dropwise, the temperature was then raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Arg(Pbf)-Thr(tBu)-NH-(D-STag) was obtained.
Retention time (Analysis Condition 1): Fmoc-Arg(Pbf)-Thr(tBu)-NH-(D-STag): 14.0 minutes; H-Arg(Pbf)-Thr(tBu)-NH-(D-STag): 12.5 minutes

### 3) Synthesis of H-Glu(OtBu)-Arg(Pbf)-Thr(tBu)-NH-(D-STag)

To the CPME solution containing H-Arg(Pbf)-Thr(tBu)-NH-(D-STag), 0.5 mL of CPME, 2 mL of DMF, 0.88 g (1.4 mmol) of Fmoc-Arg(Pbf)-OH, 0.56 g (1.3 mmol) of COMU, and 0.71 mL (4.1 mmol) of DIEPA were added, and the mixture was stirred at room temperature for 1 hour. After it was confirmed that H-Arg(Pbf)-Thr(tBu)-NH-(D-STag) was 5% or less with respect to Fmoc-Glu(OtBu)-Arg(Pbf)-Thr(tBu)-NH-(D-STag) as a product in Analysis Condition 1, 62 µL (0.5 mmol) of AEEE was added, and the mixture was stirred at room temperature for 40 minutes. The mixture was cooled to 0°C, 0.40 g (2.4 mmol) of sodium 2-mercapto-1-ethanesulfonate and 2.4 mL of DMSO were added, 0.91 mL (6.1 mmol) of DBU was added, and the mixture was stirred for 65 minutes. After it was confirmed that Fmoc-Glu(OtBu)-Arg(Pbf)-Thr(tBu)-NH-(D-STag) was 5% or less with respect to H-Glu(OtBu)-Arg(Pbf)-Thr(tBu)-NH-(D-STag) as a product in Analysis Condition 1, 13 mL of a 5% sodium carbonate aqueous solution was added dropwise, the temperature was then raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Glu(OtBu)-Arg(Pbf)-Thr(tBu)-NH-(D-STag) was obtained.
Retention time (Analysis Condition 1): Fmoc-Glu(OtBu)-Arg(Pbf)-Thr(tBu)-NH-(D-STag): 14.0 minutes; H-Glu(OtBu)-Arg(Pbf)-Thr(tBu)-NH-(D-STag): 12.6 minutes

### 4) Synthesis of H-Trp(Boc)-Glu(OtBu)-Arg(Pbf)-Thr(tBu)-NH-(D-STag)

To the CPME solution containing H-Glu(OtBu)-Arg(Pbf)-Thr(tBu)-NH-(D-STag), 0.5 mL of CPME, 2 mL of DMF, 0.71 g (1.4 mmol) of Fmoc-Trp(Boc)-OH, 0.56 g (1.3 mmol) of COMU, and 0.71 mL (4.1 mmol) of DIEPA were added, and the mixture was stirred at room temperature for 70 minutes. After it was confirmed that H-Trp(Boc)-Glu(OtBu)-Arg(Pbf)-Thr(tBu)-NH-(D-STag) was 5% or less with respect to Fmoc-Trp(Boc)-Glu(OtBu)-Arg(Pbf)-Thr(tBu)-NH-(D-STag) as a product in Analysis Condition 1, 62 µL (0.5 mmol) of AEEE was added, and the mixture was stirred at room temperature for 40 minutes. The mixture was cooled to 0°C, 0.40 g (2.4 mmol) of sodium 2-mercapto-1-ethanesulfonate and 2.4 mL of DMSO were added, 0.4 mL of DMF and 0.91 mL (6.1 mmol) of DBU were added, and the mixture was stirred for 65 minutes. After it was confirmed that Fmoc-Trp(Boc)-Glu(OtBu)-Arg(Pbf)-Thr(tBu)-NH-(D-STag) was 5% or less with respect to H-Trp(Boc)-Glu(OtBu)-Arg(Pbf)-Thr(tBu)-NH-(D-STag) as a product in Analysis Condition 1, 3.2 mL of a 1 M sulfuric acid aqueous solution was added dropwise, 13 mL of a 5% sodium carbonate aqueous solution was added, the temperature was then raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Trp(Boc)-Glu(OtBu)-Arg(Pbf)-Thr(tBu)-NH-(D-STag) was obtained.
Retention time (Analysis Condition 1): Fmoc-Trp(Boc)-Glu(OtBu)-Arg(Pbf)-Thr(tBu)-NH-(D-STag): 14.0 minutes; H-Trp(Boc)-Glu(OtBu)-Arg(Pbf)-Thr(tBu)-NH-(D-STag): 12.8 minutes

### 5) Synthesis of H-Gln(Trt)-Trp(Boc)-Glu(OtBu)-Arg(Pbf)-Thr(tBu)-NH-(D-STag)

To the CPME solution containing H-Trp(Boc)-Glu(OtBu)-Arg(Pbf)-Thr(tBu)-NH-(D-STag), 0.5 mL of CPME, 2 mL of DMF, 0.82 g (1.4 mmol) of Fmoc-Gln(Trt)-OH, 0.56 g (1.3 mmol) of COMU, and 0.71 mL (4.1 mmol) of DIEPA were added, and the mixture was stirred at room temperature for 1 hour. After it was confirmed that H-Gln(Trt)-Trp(Boc)-Glu(OtBu)-Arg(Pbf)-Thr(tBu)-NH-(D-STag) was 5% or less with respect to Fmoc-Gln(Trt)-Trp(Boc)-Glu(OtBu)-Arg(Pbf)-Thr(tBu)-NH-(D-STag) as a product in Analysis Condition 1, 62 µL (0.5 mmol) of AEEE was added, and the mixture was stirred at room temperature for 40 minutes. The mixture was cooled to 0°C, 0.40 g (2.4 mmol) of sodium 2-mercapto-1-ethanesulfonate and 3.0 mL of DMSO were added, 0.4 mL of DMF and 0.91 mL (6.1 mmol) of DBU were added, and the mixture was stirred for 65 minutes. After it was confirmed that Fmoc-Trp(Boc)-Glu(OtBu)-Arg(Pbf)-Thr(tBu)-NH-(D-STag) was 5% or less with respect to H-Gln(Trt)-Trp(Boc)-Glu(OtBu)-Arg(Pbf)-Thr(tBu)-NH-(D-STag) as a product in Analysis Condition 1, 3.2 mL of a 1 M sulfuric acid aqueous solution was added dropwise, 13 mL of a 5% sodium carbonate aqueous solution was added, the temperature was then raised to room temperature, and liquid separation was performed. To the obtained organic layer, 13 mL of 20% saline, 4 mL of a 5% sodium carbonate aqueous solution, and 0.9 mL of DMF were added, and liquid separation was performed. A CPME solution containing H-Gln(Trt)-Trp(Boc)-Glu(OtBu)-Arg(Pbf)-Thr(tBu)-NH-(D-STag) was obtained.

The obtained CPME solution was concentrated under reduced pressure, 24 mL of MeCN, 18 mL of IPA, 6 mL of CPME, and 12 mL of water were added to the residue, the precipitated solid was collected by filtration, and the obtained solid was dried under reduced pressure at 30°C. 1.84 g of H-Gln(Trt)-Trp(Boc)-Glu(OtBu)-Arg(Pbf)-Thr(tBu)-NH-(D-STag) was obtained.
Retention time (Analysis Condition 1): Fmoc-Gln(Trt)-Trp(Boc)-Glu(OtBu)-Arg(Pbf)-Thr(tBu)-NH-(D-STag): 14.2 minutes; H-Gln(Trt)-Trp(Boc)-Glu(OtBu)-Arg(Pbf)-Thr(tBu)-NH-(D-STag): 12.8 minutes

### 6) Synthesis of H-Gln-Trp-Glu-Arg-Thr-NH₂

To 1.14 g (0.50 mmol) of H-Gln(Trt)-Trp(Boc)-Glu(OtBu)-Arg(Pbf)-Thr(tBu)-NH-(D-STag), 9.5 mL of trifluoroacetic acid, 0.25 mL of water, and 0.25 mL of triisopropylsilane were added, and the mixture was stirred at room temperature for 2 hours. The reaction solution was cooled to 0°C, 70 mL of MTBE was slowly added dropwise, and a precipitate was collected by filtration. The precipitate collected by filtration was washed with 10 mL of MTBE three times, and then the precipitate was dried under reduced pressure to obtain 227 mg of H-Gln-Trp-Glu-Arg-Thr-NH₂. As a result of analyzing the obtained H-Gln-Trp-Glu-Arg-Thr-NH₂ in Analysis Condition 1, the purity was 71.0%, and D-STag and the D-STag-derived compound produced by treating D-STag with trifluoroacetic acid were not detected.
Retention time (Analysis Condition 1): H-Gln-Trp-Glu-Arg-Thr-NH₂: 0.9 minutes

### Example 6

To 0.22 g (0.2 mmol) of Fmoc-NH-(D-STag), 3.2 mL of trifluoroacetic acid, 0.09 mL of water, 0.09 mL of triisopropylsilane, and 0.18 mL of 3,6-dioxa-1,8-octanedithiol were added, and the mixture was stirred at room temperature for 2 hours. The reaction solution was analyzed in Analysis Condition 1 and Analysis Condition 2 to detect the D-STag-derived compound.
Retention time (Analysis Condition 1): 11.8 minutes; 11.9 minutes; 12.6 minutes; 13.0 minutes
Retention time (Analysis Condition 2): 0.5 minutes; 1.4 minutes; 2.0 minutes; 2.5 minutes

Similarly to Example 3, it was found that also in D-STag, the D-STag-derived compound by-produced under conditions after deprotection such as D-STag can be detected with good separation performance even in any of Analysis Conditions 1 and 2.

### Example 7

### Synthesis of H-Gln-Trp-Glu-Arg-Thr-NH₂

### 1) Synthesis of H-Thr(tBu)-NH-(X-STag)

After 1.11 g (1.0 mmol) of Fmoc-NH-(X-STag) was dissolved in 8 mL of CPME and 2 mL of DMF and cooled to 0°C, 0.33 mL (1.9 mmol) of DIEPA and 0.30 g (1.8 mmol) of sodium 2-mercapto-1-ethanesulfonate were added, and 0.67 mL (4.5 mmol) of DBU was added dropwise thereto. After the mixture was stirred at 0°C for 1 hour and it was confirmed that Fmoc-NH-(X-STag) was 5% or less with respect to NH₂-(X-STag) as a product in Analysis Condition 1 and Analysis Condition 2, 2.4 mL of a 1 M sulfuric acid aqueous solution was added dropwise, 9 mL of a 5% sodium carbonate aqueous solution was added, the temperature was then raised to room temperature, and liquid separation was performed. To the obtained organic layer, 23 mL of 20% saline, 8 mL of a 5% sodium carbonate aqueous solution, and 1.6 mL of DMF were added, and liquid separation was performed to obtain a CPME solution containing NH₂-(X-STag).

To the CPME solution containing NH₂-(X-STag), 0.5 mL of CPME, 2 mL of DMF, 0.54 g (1.4 mmol) of Fmoc-Thr(tBu)-OH, 0.56 g (1.3 mmol) of COMU, and 0.71 mL (4.1 mmol) of DIEPA were added, and the mixture was stirred at room temperature for 110 minutes. After it was confirmed that NH₂-(X-STag) was 5% or less with respect to Fmoc-Thr(tBu)-NH-(X-STag) as a product in Analysis Condition 1 and Analysis Condition 2, 0.15 g (1.4 mmol) of aminomethanephosphonic acid and 2.4 mL of DMSO were added, and the mixture was stirred at room temperature for 40 minutes. The mixture was cooled to 0°C, 0.40 g (2.4 mmol) of sodium 2-mercapto-1-ethanesulfonate was added, 0.91 mL (6.1 mmol) of DBU was added, and the mixture was stirred for 100 minutes. After it was confirmed that Fmoc-Thr(tBu)-NH-(X-STag) was 5% or less with respect to H-Thr(tBu)-NH-(X-STag) as a product in Analysis Condition 1 and Analysis Condition 2, 20 mL of a 5% potassium hydrogen carbonate aqueous solution was added dropwise, the temperature was then raised to room temperature, and liquid separation was performed. To the obtained organic layer, 15 mL of 20% saline and 1.6 mL of DMF were added, and liquid separation was performed. A CPME solution containing H-Thr(tBu)-NH-(X-STag) was obtained.
Retention time (Analysis Condition 1): Fmoc-NH-(X-STag): 15.0 minutes; NH₂-(X-STag): 14.6 minutes; Fmoc-Thr(tBu)-NH-(X-STag): 14.8 minutes; H-Thr(tBu)-NH-(X-STag): 12.5 minutes
Retention time (Analysis Condition 2): Fmoc-NH-(X-STag): 3.2 minutes; NH₂-(X-STag): 2.0 minutes; Fmoc-Thr(tBu)-NH-(X-STag): 3.6 minutes; H-Thr(tBu)-NH-(X-STag): 5.0 minutes

### 2) Synthesis of H-Arg(Pbf)-Thr(tBu)-NH-(X-STag)

To the CPME solution containing H-Thr(tBu)-NH-(X-STag), 0.6 mL of CPME, 2 mL of DMF, 0.88 g (1.4 mmol) of Fmoc-Arg(Pbf)-OH, 0.56 g (1.3 mmol) of COMU, and 0.47 mL (2.7 mmol) of DIEPA were added, and the mixture was stirred at room temperature for 1 hour. After it was confirmed that H-Thr(tBu)-NH-(X-STag) was 5% or less with respect to Fmoc-Arg(Pbf)-Thr(tBu)-NH-(X-STag) as a product in Analysis Condition 1 and Analysis Condition 2, 81 µL (1.4 mmol) of 2-aminoethanol (AE) was added, and the mixture was stirred at room temperature for 40 minutes. The mixture was cooled to 0°C, to which 0.40 g (2.4 mmol) of sodium 2-mercapto-1-ethanesulfonate and 2.4 mL of DMSO were added, and then thereto 0.91 mL (6.1 mmol) of DBU was added, and the mixture was stirred for 90 minutes. After it was confirmed that Fmoc-Arg(Pbf)-Thr(tBu)-NH-(X-STag) was 5% or less with respect to H-Arg(Pbf)-Thr(tBu)-NH-(X-STag) as a product in Analysis Condition 1 and Analysis Condition 2, 13 mL of a 5% potassium hydrogen carbonate aqueous solution was added thereto dropwise, the mixture was then warmed to room temperature, and liquid separation was performed. To the obtained organic layer, 13 mL of 20% saline and 0.9 mL of DMF were added, and liquid separation was performed. A CPME solution containing H-Arg(Pbf)-Thr(tBu)-NH-(X-STag) was obtained.
Retention time (Analysis Condition 1): Fmoc-Arg(Pbf)-Thr(tBu)-NH-(X-STag): 14.1 minutes; H-Arg(Pbf)-Thr(tBu)-NH-(X-STag): 12.6 minutes
Retention time (Analysis Condition 2): Fmoc-Arg(Pbf)-Thr(tBu)-NH-(X-STag): 7.0 minutes; H-Arg(Pbf)-Thr(tBu)-NH-(X-STag): 7.8 minutes

### 3) Synthesis of H-Glu(OtBu)-Arg(Pbf)-Thr(tBu)-NH-(X-STag)

To the CPME solution containing H-Arg(Pbf)-Thr(tBu)-NH-(X-STag), 0.5 mL of CPME, 2 mL of DMF, 0.57 g (1.4 mmol) of Fmoc-Glu(OtBu)-OH, 0.56 g (1.3 mmol) of COMU, and 0.71 mL (4.1 mmol) of DIEPA were added, and the mixture was stirred at room temperature for 75 minutes. After it was confirmed that H-Arg(Pbf)-Thr(tBu)-NH-(X-STag) was 5% or less with respect to Fmoc-Glu(OtBu)-Arg(Pbf)-Thr(tBu)-NH-(X-STag) as a product in Analysis Condition 1 and Analysis Condition 2, 0.15 g (1.4 mmol) of aminomethanephosphonic acid and 2.4 mL of DMSO were added thereto, and the mixture was stirred at room temperature for 105 minutes. The mixture was cooled to 0°C, 0.40 g (2.4 mmol) of sodium 2-mercapto-1-ethanesulfonate was added, 0.91 mL (6.1 mmol) of DBU was added, and the mixture was stirred for 85 minutes. After it was confirmed that Fmoc-Glu(OtBu)-Arg(Pbf)-Thr(tBu)-NH-(X-STag) was 5% or less with respect to H-Glu(OtBu)-Arg(Pbf)-Thr(tBu)-NH-(X-STag) as a product in Analysis Condition 1 and Analysis Condition 2, 16 mL of a 5% potassium hydrogen carbonate aqueous solution was added dropwise, the temperature was then raised to room temperature, and liquid separation was performed. To the obtained organic layer, 13 mL of 20% saline and 0.9 mL of DMF were added, and liquid separation was performed. A CPME solution containing H-Glu(OtBu)-Arg(Pbf)-Thr(tBu)-NH-(X-STag) was obtained.
Retention time (Analysis Condition 1): Fmoc-Glu(OtBu)-Arg(Pbf)-Thr(tBu)-NH-(X-STag): 14.1 minutes; H-Glu(OtBu)-Arg(Pbf)-Thr(tBu)-NH-(X-STag): 12.6 minutes
Retention time (Analysis Condition 2): Fmoc-Glu(OtBu)-Arg(Pbf)-Thr(tBu)-NH-(X-STag): 7.0 minutes; H-Glu(OtBu)-Arg(Pbf)-Thr(tBu)-NH-(X-STag): 7.6 minutes

### 4) Synthesis of H-Trp(Boc)-Glu(OtBu)-Arg(Pbf)-Thr(tBu)-NH-(X-STag)

To the CPME solution containing H-Glu(OtBu)-Arg(Pbf)-Thr(tBu)-NH-(X-STag), 0.5 mL of CPME, 2 mL of DMF, 0.71 g (1.4 mmol) of Fmoc-Trp(Boc)-OH, 0.56 g (1.3 mmol) of COMU, and 0.71 mL (4.1 mmol) of DIEPA were added, and the mixture was stirred at room temperature for 75 minutes. After it was confirmed that H-Trp(Boc)-Glu(OtBu)-Arg(Pbf)-Thr(tBu)-NH-(X-STag) was 5% or less with respect to Fmoc-Trp(Boc)-Glu(OtBu)-Arg(Pbf)-Thr(tBu)-NH-(X-STag) as a product in Analysis Condition 1 and Analysis Condition 2, 0.19 g (1.4 mmol) of 3-aminopropanesulfonic acid and 2.4 mL of DMSO were added, and the mixture was stirred at room temperature for 30 minutes. The mixture was cooled to 0°C, 0.40 g (2.4 mmol) of sodium 2-mercapto-1-ethanesulfonate was added, 0.91 mL (6.1 mmol) of DBU was added, and the mixture was stirred for 100 minutes. After it was confirmed that Fmoc-Glu(OtBu)-Arg(Pbf)-Thr(tBu)-NH-(X-STag) was 5% or less with respect to H-Trp(Boc)-Glu(OtBu)-Arg(Pbf)-Thr(tBu)-NH-(X-STag) as a product in Analysis Condition 1 and Analysis Condition 2, 16 mL of a 5% potassium hydrogen carbonate aqueous solution was added, the temperature was then raised to room temperature, and liquid separation was performed. To the obtained organic layer, 13 mL of 20% saline and 0.9 mL of DMF were added, and liquid separation was performed. A CPME solution containing H-Trp(Boc)-Glu(OtBu)-Arg(Pbf)-Thr(tBu)-NH-(X-STag) was obtained.
Retention time (Analysis Condition 1): Fmoc-Trp(Boc)-Glu(OtBu)-Arg(Pbf)-Thr(tBu)-NH-(X-STag): 14.2 minutes; H-Trp(Boc)-Glu(OtBu)-Arg(Pbf)-Thr(tBu)-NH-(X-STag): 12.8 minutes
Retention time (Analysis Condition 2): Fmoc-Trp(Boc)-Glu(OtBu)-Arg(Pbf)-Thr(tBu)-NH-(X-STag): 7.1 minutes; H-Trp(Boc)-Glu(OtBu)-Arg(Pbf)-Thr(tBu)-NH-(X-STag): 7.6 minutes

### 5) Synthesis of H-Gln(Trt)-Trp(Boc)-Glu(OtBu)-Arg(Pbf)-Thr(tBu)-NH-(X-STag)

0.47 g (corresponding to 0.2 mmol) of the residue was weighed from 2.36 g of the residue obtained by concentrating the CPME solution containing H-Trp(Boc)-Glu(OtBu)-Arg(Pbf)-Thr(tBu)-NH-(X-STag), 1.8 mL of 2-methyltetrahydrofuran, 0.4 mL of DMF, 0.17 g (0.3 mmol) of Fmoc-Gln(Trt)-OH, 0.11 g (0.3 mmol) of COMU, and 0.14 mL (0.8 mmol) of DIEPA were added, and the mixture was stirred at room temperature for 75 minutes. After it was confirmed that H-Trp(Boc)-Glu(OtBu)-Arg(Pbf)-Thr(tBu)-NH-(X-STag) was 5% or less with respect to Fmoc-Gln(Trt)-Trp(Boc)-Glu(OtBu)-Arg(Pbf)-Thr(tBu)-NH-(X-STag) as a product in Analysis Condition 1 and Analysis Condition 2, 38 mg (0.3 mmol) of 2-aminoethyl hydrogen sulfate and 0.6 mL of DMSO were added, and the mixture was stirred at room temperature for 30 minutes. The mixture was cooled to 0°C, 76 mg (0.5 mmol) of 3-mercapto-1-propanephosphonic acid (synthesized in Reference Example 1) was added, 0.18 mL (1.2 mmol) of DBU was added, and the mixture was stirred for 2 hours. After it was confirmed that Fmoc-Gln(Trt)-Trp(Boc)-Glu(OtBu)-Arg(Pbf)-Thr(tBu)-NH-(X-STag) was 5% or less with respect to H-Gln(Trt)-Trp(Boc)-Glu(OtBu)-Arg(Pbf)-Thr(tBu)-NH-(X-STag) as a product in Analysis Condition 1 and Analysis Condition 2, 5 mL of a 5% potassium hydrogen carbonate aqueous solution, 1 mL of CPME, and 1 mL of THF were added, the temperature was then raised to room temperature, and liquid separation was performed. To the obtained organic layer, 3 mL of 20% saline and 0.2 mL of DMF were added, and liquid separation was performed. A CPME solution containing H-Gln(Trt)-Trp(Boc)-Glu(OtBu)-Arg(Pbf)-Thr(tBu)-NH-(X-STag) was obtained.

The obtained CPME solution was concentrated under reduced pressure, 4 mL of MeCN and 1 mL of IPA were added to the residue, the precipitated solid was collected by filtration, and the obtained solid was dried under reduced pressure. 0.31 g of H-Gln(Trt)-Trp(Boc)-Glu(OtBu)-Arg(Pbf)-Thr(tBu)-NH-(X-STag) was obtained.
Retention time (Analysis Condition 1): Fmoc-Gln(Trt)-Trp(Boc)-Glu(OtBu)-Arg(Pbf)-Thr(tBu)-NH-(X-STag): 14.9 minutes; H-Gln(Trt)-Trp(Boc)-Glu(OtBu)-Arg(Pbf)-Thr(tBu)-NH-(X-STag): 12.9 minutes
Retention time (Analysis Condition 2): Fmoc-Gln(Trt)-Trp(Boc)-Glu(OtBu)-Arg(Pbf)-Thr(tBu)-NH-(X-STag): 7.4 minutes; H-Gln(Trt)-Trp(Boc)-Glu(OtBu)-Arg(Pbf)-Thr(tBu)-NH-(X-STag): 7.8 minutes

### 6) Synthesis of H-Gln-Trp-Glu-Arg-Thr-NH₂

To 0.25 g (0.11 mmol) of H-Gln(Trt)-Trp(Boc)-Glu(OtBu)-Arg(Pbf)-Thr(tBu)-NH-(X-STag), 3.2 mL of trifluoroacetic acid, 0.09 mL of water, 0.09 mL of triisopropylsilane, and 0.18 mL of 3,6-dioxa-1,8-octanedithiol were added, and the mixture was stirred at room temperature for 2 hours. The reaction solution was cooled to 0°C, 30 mL of MTBE was slowly added dropwise, and a precipitate was collected by filtration. The precipitate collected by filtration was washed with 5 mL of MTBE three times, and then the precipitate was dried under reduced pressure to obtain 78 mg of H-Gln-Trp-Glu-Arg-Thr-NH₂. As a result of analyzing the obtained H-Gln-Trp-Glu-Arg-Thr-NH₂ in Analysis Condition 1, the purity was 82.0%, X-STag was not detected, and 0.2% of the X-STag-derived compound produced by treating X-STag with trifluoroacetic acid was detected.
Retention time (Analysis Condition 1): H-Gln-Trp-Glu-Arg-Thr-NH₂: 0.9 minutes

### Example 8

To 0.22 g (0.2 mmol) of Fmoc-NH-(X-STag), 3.2 mL of trifluoroacetic acid, 0.09 mL of water, 0.09 mL of triisopropylsilane, and 0.18 mL of 3,6-dioxa-1,8-octanedithiol were added, and the mixture was stirred at room temperature for 2 hours. The reaction solution was analyzed in Analysis Condition 1 and Analysis Condition 2 to detect the X-STag-derived compound.
Retention time (Analysis Condition 1): 11.4 minutes; 12.0 minutes; 12.3 minutes; 12.7 minutes; 13.4 minutes
Retention time (Analysis Condition 2): 0.5 minutes; 2.4 minutes; 2.8 minutes; 4.1 minutes; 4.9 minutes

In Example 7, it was found that since the target peptide was crystallized using MTBE and separated and purified after deprotection of, for example, X-STag, only a small amount of the X-STag-derived compound was detected; however, the X-STag-derived compound in the mixed liquid before the crystallization operation can be detected with good separation performance using any of Analysis Conditions 1 and 2.

### Reference Example 1

### Synthesis of 3-mercapto-1-propanephosphonic acid

777 mg (3 mmol) of diethyl 3-bromo-1-propanephosphonate was dissolved in 6 mL of acetonitrile, 1.66 g (6 mmol) of triphenylmethanethiol and 0.54 mL (3.9 mmol) of triethylamine were added, and the mixture was stirred at 50°C for 18 hours. 20 mL of MTBE and 20 mL of water were added to perform liquid separation, and the organic layer was washed with a 20% sodium chloride aqueous solution. The obtained organic layer was concentrated to obtain 2.39 g of a concentrated residue. The residue was dissolved in heptane/ethyl acetate (2/1) and purified by silica gel column chromatography to obtain 1.36 g of diethyl 3-(triphenylmethylthio)-1-propanephosphonate in quantitative yield.

1.36 g of the diethyl 3-(triphenylmethylthio)-1-propanephosphonate obtained above was dissolved in 10 mL of acetonitrile, 1.95 mL (15 mmol) of trimethylsilyl bromide was added, and the mixture was stirred at room temperature for 18 hours. 4 mL of methanol was added, and the mixture was reacted with excessive trimethylsilyl bromide and then concentrated under reduced pressure. 15 mL of acetonitrile was added to the concentrated residue, and a solid that was not dissolved was separated by filtration. The filtrate was concentrated to obtain 2.32 g of a concentrated residue. 22.8 mL of trifluoroacetic acid, 0.6 mL of water, and 0.6 mL of triisopropylsilane were added to the obtained residue, and the mixture was stirred at room temperature for 2 hours. 24 mL of MTBE was added to the reaction solution, the precipitated solid was separated by filtration, and then the filtrate was concentrated to obtain 3.30 g of a concentrated residue. 35 mL of MTBE, 12 mL of heptane, and 10 mL of water were added to the residue to perform liquid separation. The organic layer was reextracted twice with 5 mL of water, and then the aqueous layer was concentrated together under reduced pressure. 0.72 g of a concentrated residue was obtained. The residue was dissolved in water and purified by HPLC column chromatography (column: ODS-AQ-HG manufactured by YMC; 10 mm × 250 mm) to obtain 0.30 g of target 3-mercapto-1-propanephosphonic acid.
¹H-NMR (DMSO-d₆, 500 MHz)σ = 1.60(2H,m), 1.71(2H,m), 2.32(1H,br), 2.53(2H,m)
LC-MS m/z 157.00[M+H⁺]

### Comparative Example 1

0.79 g (1.0 mmol) of B-STag was dissolved in 2 mL of CPME and 3 mL of THF, 1.06 g (2.5 mmol) of Fmoc-Glu(OtBu)-OH, 0.48 g (2.5 mmol) of WSCI·HCl, and 12.2 mg (0.1 mmol) of 4-dimethylaminopyridine were added, and the mixture was stirred at room temperature for 2 hours. As a result of analysis in Analysis Condition 3, neither B-STag nor Fmoc-Glu(OtBu)-O-(B-STag) as a product was detected.

### Comparative Example 2

After 1.09 g (1.0 mmol) of Fmoc-NH-(D-STag) was dissolved in 9 mL of CPME and 2 mL of DMF and cooled to 0°C, 0.32 mL (1.9 mmol) of DIEPA and 0.30 g (1.8 mmol) of sodium 2-mercapto-1-ethanesulfonate were added, and 0.67 mL (4.5 mmol) of DBU was added dropwise thereto. The mixture was stirred at 0°C for 1 hour and analyzed in Analysis Condition 3, and as a result, neither Fmoc-NH-(D-STag) nor NH₂-(D-STag) as a product was detected.

### Comparative Example 3

After 1.11 g (1.0 mmol) of Fmoc-NH-(X-STag) was dissolved in 8 mL of CPME and 2 mL of DMF and cooled to 0°C, 0.33 mL (1.9 mmol) of DIEPA and 0.30 g (1.8 mmol) of sodium 2-mercapto-1-ethanesulfonate were added, and 0.67 mL (4.5 mmol) of DBU was added dropwise thereto. The mixture was stirred at 0°C for 1 hour and analyzed in Analysis Condition 3, and as a result, neither Fmoc-NH-(X-STag) nor NH₂-(X-STag) as a product was detected.

As shown in Comparative Examples 1 to 3, in Analysis Condition 3, none of B-STag, D-STag, and X-STag could be detected regardless of the presence or absence of Fmoc bond. Therefore, in the HPLC measurement system using acetonitrile, these carrier for liquid phase peptide synthesis itself and the compound in which the carrier for liquid phase peptide synthesis and the amino acid, peptide, or amino acid amide are bound cannot be detected, and the carrier for liquid phase peptide synthesis and impurities derived from the carrier for liquid phase peptide synthesis cannot also be simultaneously analyzed and detected with the target peptide or final target peptide. On the other hand, as shown in Examples 1 to 8, in Analysis Conditions 1 and 2 using isopropanol as an eluent, the carrier for liquid phase peptide synthesis itself and the compound in which the carrier for liquid phase peptide synthesis and the amino acid, peptide, or amino acid amide are bound could be detected, and the target peptide or final target peptide and impurities derived from the carrier for liquid phase peptide synthesis could also be analyzed and detected.

## Claims

1. A method for simultaneously analyzing one or more compounds selected from the group consisting of a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound, with a target peptide or final target peptide, the analysis method using high-performance liquid chromatography or supercritical fluid chromatography using, as an eluent, a solution containing an alcohol having 1 to 4 carbon atoms,
wherein the carrier for liquid phase peptide synthesis being a compound of Formula (I) below: 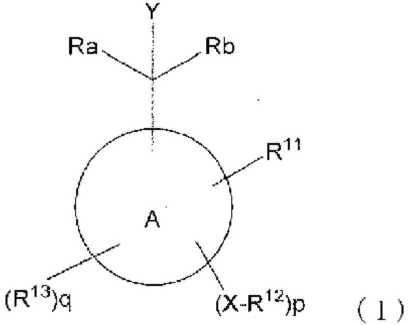
wherein ring A represents a C4-20 aromatic ring which may contain a hetero atom and may be polycyclic;
R¹¹ is a hydrogen atom, or when ring A is a benzene ring and Rb is a group of Formula (a) below, represents a single bond together with R¹⁴, and may form a fluorene ring together with ring A and ring B or may form a xanthene ring together with ring A and ring B via an oxygen atom;
p X's each independently represent -O-, -S-,-C(=O)O-, -C(=O)NH-, -NHC(=O)-, or -NR¹⁷- (R¹⁷ represents a hydrogen atom, an alkyl group, or an aralkyl group); and
p R¹²'s each independently represent an organic group of an aliphatic hydrocarbon group substituted with an aliphatic hydrocarbon group via an oxygen atom, or Formula (b) ;
-R²⁰-X³-D (b)
provided that R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound;
q R¹³'s each independently are a hydrogen atom or represent an organic group having an aliphatic hydrocarbon group which may be substituted with a silyl group or an aliphatic hydrocarbon group via an oxygen atom;
p and q each represent an integer of 0 to 4 and p + q is 1 or more and 4 or less;
ring A may further have, in addition to p XR¹²'s, a substituent selected from the group consisting of a halogen atom, a C1-6 alkyl group which may be substituted with a halogen atom, and a C1-6 alkoxy group which may be substituted with a halogen atom;
Ra represents a hydrogen atom or an aromatic ring which may be substituted with a halogen atom; and
Rb represents a hydrogen atom, an aromatic ring which may be substituted with a halogen atom, or a group of Formula (a): 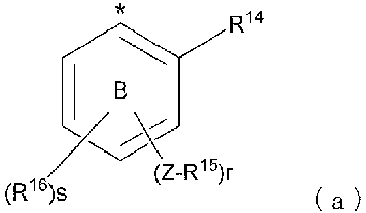
wherein * represents a bonding position;
r and s each represent an integer of 0 to 4 and r + s is 4 or less;
r Z's each independently represent -O-, -S-,-C(=O)O-, -C(=O)NH-, -NHC(=O)-, or -NR¹⁸- (R¹⁸ represents a hydrogen atom, an alkyl group, or an aralkyl group); and
r R¹⁵'s independently represent an organic group of an aliphatic hydrocarbon group substituted with an aliphatic hydrocarbon group, or Formula (b);
-R²⁰-X³-D (b)
provided that R²⁰ represents a linear or branched alkylene group having 6 to 16 carbon atoms, X³ represents an oxygen atom or -C(=O)NR²¹- (R²¹ represents a hydrogen atom or an alkyl group having 1 to 4 carbon atoms), and D represents any of a silyl group or an alkyl group to which a silyloxy group is bound;
s R¹⁶'s each independently represent an organic group having an aliphatic hydrocarbon group which may be substituted with a silyl group or an aliphatic hydrocarbon group via an oxygen atom;
R¹⁴ represents a hydrogen atom, or represents a single bond together with R¹¹, and may form a fluorene ring together with ring A and ring B, or may form a xanthene ring together with ring A and ring B via an oxygen atom; and
ring B may further have, in addition to r ZR¹⁵'s, a substituent selected from the group consisting of a halogen atom, a C1-6 alkyl group which may be substituted with a halogen atom, and a C1-6 alkoxy group which may be substituted with a halogen atom; and
Y represents a hydroxy group, a thiol group, NHR¹⁹ (R¹⁹ represents a hydrogen atom, an alkyl group, or an aralkyl group), or a halogen atom.

2. A method for simultaneously analyzing one or more compounds selected from the group consisting of a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound, with a target peptide or final target peptide, the analysis method using high-performance liquid chromatography or supercritical fluid chromatography using, as an eluent, a solution containing an alcohol having 1 to 4 carbon atoms,
wherein the carrier for liquid phase peptide synthesis being a compound of Formula (1), (13), or (14) below: 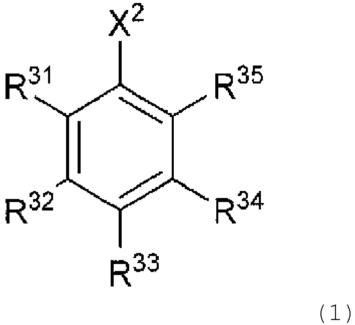
wherein, X² represents -CH₂OR⁴⁴ (R⁴⁴ represents a hydrogen atom, a halogenocarbonyl group, an active ester-type carbonyl group, or an active ester-type sulfonyl group), -CH₂NHR⁴⁵ (R⁴⁵ represents a hydrogen atom, a linear or branched alkyl group having 1 to 6 carbon atoms, or an aralkyl group), a halogenomethyl group, a formyl group, or an oxime, and at least one of R³¹, R³², R³³, R³⁴, and R³⁵ represents a group of Formula (c),
-O-R²⁰-X³-D (c)
and the rest represents a hydrogen atom, a halogen atom, an alkyl group having 1 to 4 carbon atoms, or an alkoxy group having 1 to 4 carbon atoms;
R²⁰ represents a linear or branched alkylene group having 6 to 16 carbon atoms;
X³ represents O or CONR²¹ (R²¹ represents a hydrogen atom or an alkyl group having 1 to 4 carbon atoms); and
D represents a group of Formula (2), (3), (4), (5), (6), (7), (8), (9), (10), (11), or (12): 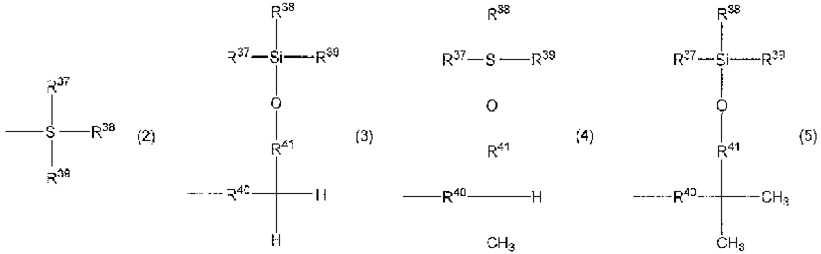 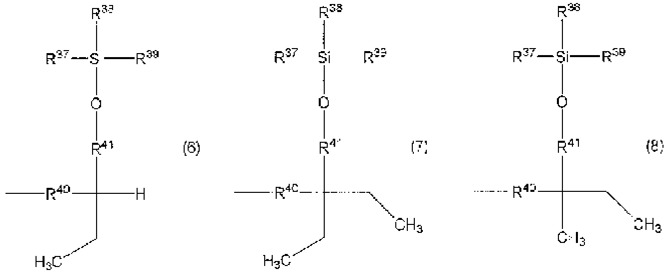 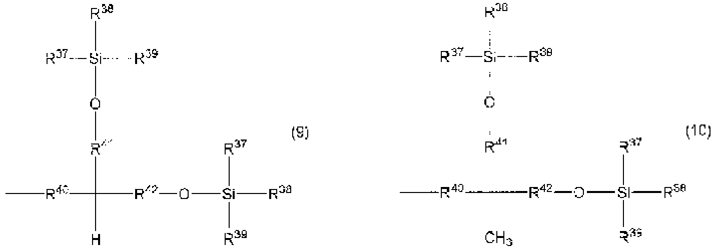 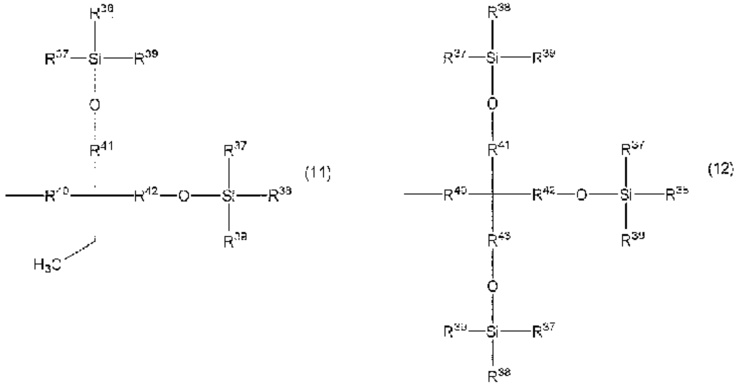
wherein R³⁷, R³⁸, and R³⁹ are the same or different and each represent a linear or branched alkyl group having 1 to 6 carbon atoms or an aryl group which may have a substituent; R⁴⁰ represents a single bond or a linear or branched alkylene group having 1 to 3 carbon atoms; and R⁴¹, R⁴², and R⁴³ each represent a linear or branched alkylene group having 1 to 3 carbon atoms, 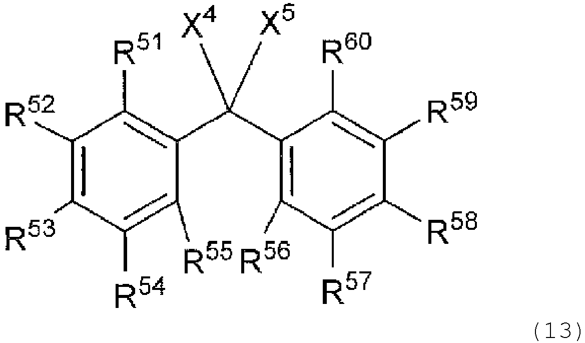
wherein X⁴ represents -OR⁶¹ (R⁶¹ represents a hydrogen atom, an active ester-type carbonyl group, or an active ester-type sulfonyl group), -NHR⁴⁵, azide, halogen, isocyanate, or =N-OH or =O together with X⁵, when X⁴ is-OR⁶¹, -NHR⁴⁵, azide, or halogen, X⁵ represents a hydrogen atom, a linear or branched alkyl group or alkenyl group having 1 to 4 carbon atoms, or a cycloalkyl group, and when X⁴ is isocyanate, X⁵ represents a linear or branched alkyl group or alkenyl group having 1 to 4 carbon atoms, or a cycloalkyl group;
at least one of R⁵¹ to R⁶⁰ represents a group of Formula (c), and the rest represents a hydrogen atom, a halogen atom, an alkyl group having 1 to 4 carbon atoms, or an alkoxy group having 1 to 4 carbon atoms; and
when X⁴ is -OR⁶¹, -NHR⁴⁵, azide, or halogen and X⁵ is a hydrogen atom, or when X⁴ is =O together with X⁵, R⁵⁵ and R⁵⁶ may be bound via an oxygen atom to form a xanthene ring; 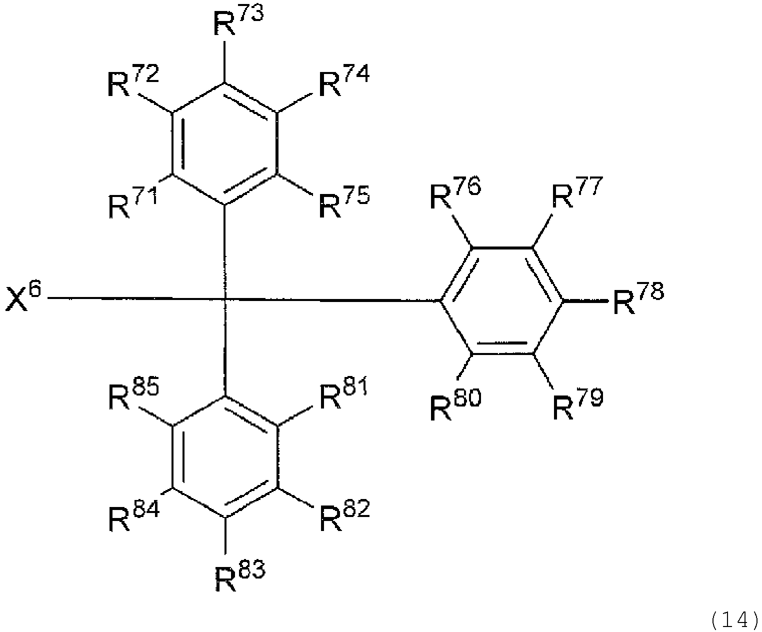
wherein X⁶ represents a hydroxy group or a halogen atom, at least one of R⁷¹ to R⁸⁵ represents a group of Formula (c), the rest represents a hydrogen atom, a halogen atom, an alkyl group having 1 to 4 carbon atoms, or an alkoxy group having 1 to 4 carbon atoms, and R⁸⁰ and R⁸¹ may be bound by a single bond to form a fluorene ring, or may be bound via an oxygen atom to form a xanthene ring.

3. A method for simultaneously analyzing one or more compounds selected from the group consisting of a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound, with a target peptide or final target peptide, the analysis method using high-performance liquid chromatography or supercritical fluid chromatography using, as an eluent, a solution containing an alcohol having 1 to 4 carbon atoms,
wherein the carrier for liquid phase peptide synthesis being a compound of Formula (20) or (21) below: 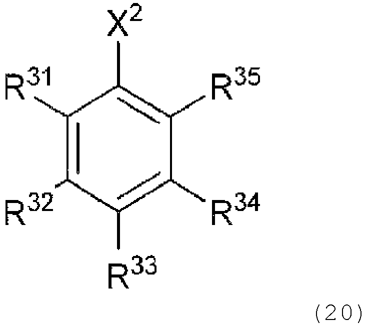
wherein X² represents -CH₂OR⁴⁴ (R⁴⁴ represents a hydrogen atom, a halogenocarbonyl group, an active ester-type carbonyl group, or an active ester-type sulfonyl group), -CH₂NHR⁴⁵ (R⁴⁵ represents a hydrogen atom, a linear or branched alkyl group having 1 to 6 carbon atoms, or an aralkyl group), a halogenomethyl group, a formyl group, or an oxime, and at least one of R³¹, R³², R³³, R³⁴, and R³⁵ represents a group of Formula (d),
-O-R²⁰-X³-E (d)
and the rest represents a hydrogen atom, a halogen atom, an alkyl group having 1 to 4 carbon atoms, or an alkoxy group having 1 to 4 carbon atoms;
R²⁰ represents a linear or branched alkyl group or alkylene group having 6 to 30 carbon atoms;
X³ is absent or represents an oxygen atom; and
E is absent or represents a linear or branched alkylene group having 6 to 30 carbon atoms: 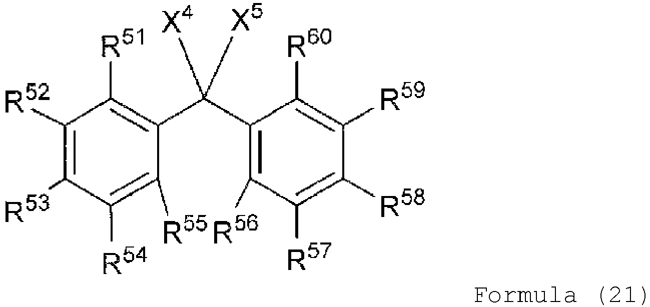
wherein X⁴ represents a hydroxyl group or -NHR⁹⁰ (R⁹⁰ represents a hydrogen atom, a linear or branched alkyl group having 1 to 6 carbon atoms, or an aralkyl group), and X⁵ represents a hydrogen atom or a phenyl group which may be substituted with a halogen atom; and
at least one of R⁵¹ to R⁶⁰ represents a group of Formula (d), and the rest represents a hydrogen atom, a halogen atom, an alkyl group having 1 to 4 carbon atoms, or an alkoxy group having 1 to 4 carbon atoms.

4. The analysis method according to any one of claims 1 to 3, wherein the carrier for liquid phase peptide synthesis is a compound which is bound to an amino acid, a peptide, or an amino acid amide directly or via a linker to make the amino acid, the peptide, or the amino acid amide soluble in an organic solvent and insoluble in water.

5. The analysis method according to any one of claims 1 to 4, wherein a sample used for analysis is a reaction solution in which an object to be analyzed is not subjected to solid-liquid separation.

6. The analysis method according to any one of claims 1 to 4, wherein a sample used for analysis is a sample obtained after subjecting an object to be analyzed to solid-liquid separation or purification by chromatography.

7. The method according to any one of claims 1 to 6, wherein one or more detected or undetected compounds selected from the group consisting of a carrier for liquid phase peptide synthesis, a component derived from the carrier for liquid phase peptide synthesis that is by-produced under conditions after deprotection of the carrier for liquid phase peptide synthesis, an amino acid to which the carrier for liquid phase peptide synthesis is bound, and a peptide compound to which the carrier for liquid phase peptide synthesis is bound are linked with a detected target peptide or a detected final target peptide to perform evaluation.
